# FASCICULE DE BREVET EUROPEEN

(11) **EP 1 709 018 B1**
(45) Date de publication et mention de la délivrance du brevet: **20.07.2011**
(21) Numéro de dépôt: 05717377.5
(22) Date de dépôt: 07.01.2005
(51) Int. Cl.: C07D 277/36, C07D 277/56, C07D 417/04, C07D 417/12, A61K 31/426, A61K 31/427, A61P 25/28

(54) **DERIVES D'ACYLAMINOTHIAZOLE ET LEUR APPLICATION COMME INHIBITEURS DE BETA-AMYLOIDE**
ACYLAMINOTHIAZOLDERIVATE UND DEREN VERWENDUNG ALS BETA-AMYLOID-INHIBITOREN
ACYLAMINOTHIAZOLE DERIVATIVES AND USE THEREOF AS BETA-AMYLOID INHIBITORS

(30) Priorité: 16.01.2004 FR 0400388; 22.07.2004 FR 0408116
(43) Date de publication de la demande: 11.10.2006
(73) Titulaire: Sanofi-Aventis, 75013 Paris (FR)
(72) Inventeur: BALTZER, Sylvie, F-67200 Strasbourg (FR); PASCAL, Marc, F-32000 Pujaudran (FR); VAN DORSSELAER, Viviane, F-67100 Strasbourg (FR)
(74) Mandataire: Lecocq, Fabrice
(86) Numéro de dépôt international: PCT/FR2005/000029
(87) Numéro de publication internationale: WO 2005/073202

(56) Documents cités:
- WO-A-03/014095
- WO-A-2004/009565
- WO-A-2004/033439

## Description

La présente invention a pour objet des dérivés d'acylaminothiazole, leur préparation et leur utilisation en thérapeutique.

On connaît déjà des composés dérivés d'acylaminothiazole, décrits dans les documents WO03/014095 A et WO2004/033439 A, inhibiteurs de la formation du peptide β-amyloïdé (β-A4).

Il existe toujours une nécessité de trouver et de développer des produits inhibiteurs de la formation du peptide β-amyloïde (β-A4). Les composés de l'invention répondent à ce but.

La présente invention a pour premier objet des composés répondant à la formule générale (I) : 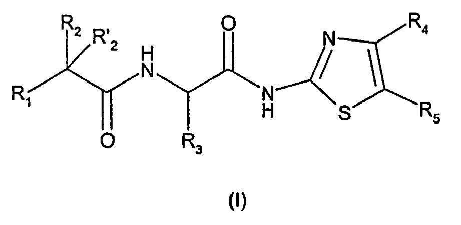 dans laquelle,
R₁ représente soit un C₁₋₆ alkyle éventuellement substitué par un à trois substituants choisis parmi un halogène, un trifluorométhyle, un hydroxy, un C₁₋₆ alcoxy, un C₁₋₆ thioalkyle, un thiophène ou un phényle ; soit un C₃₋₇ cycloalkyle, un thiophène, un benzothiophène, un pyridinyle, un furanyle ou un phényle ; les dits groupes phényle étant éventuellement substitués par un à trois substituants choisis parmi un atome d'halogène, un C₁₋₆ alkyle, un C₁₋₆ alcoxy, un hydroxy, un méthylènedioxy, un phénoxy, un benzyloxy ou un trifluorométhyle ;
R₂ et R'₂ représentent indépendamment l'un de l'autre un atome d'hydrogène, un atome d'halogène, un hydroxy, un C₁₋₃ alcoxy, un C₁₋₃ alkyle, un C₃₋₇ cycloalkyle, un groupe O-C(O)-C₁₋₆ alkyle, ou R₂ et R'₂ forment ensemble un groupe oxo ;
R₃ représente soit un atome d'hydrogène, soit un C₁₋₆ alkyle éventuellement substitué par un hydroxy, un C₁₋₆ cycloalkyle ou un C₁₋₃ alcoxy ;
R₄ et R₅ représentent, indépendamment l'un de l'autre un atome d'hydrogène, un C₁₋₇ alkyle, un trifluorométhyle, un groupe L ou un groupe Z ; 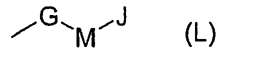 G représente un C₁₋₇ alkyle ou une liaison simple ;
M représente un C₃₋₇ cycloalkyle, un phényle, un naphtyle ou un pyridinyle, le groupe M étant éventuellement substitué par un ou plusieurs groupes choisis parmi un atome d'halogène, un groupe hydroxy, un C₁₋₃ alkyle, un C₁₋₃ alcoxy, un trifluorométhyle, un trifluorométhoxy, un -O-CHF₂ ;
J représente un atome d'hydrogène ou un groupe -Y-K ;
Y représente une liaison simple, un atome d'oxygène, de soufre, un groupe -C₁₋₄ alkylène-, -O-C₁₋₄ alkylène-, -C₁₋₄ alkylène-O- ou -N(W)-, le groupe -C₁₋₄ alkylène- étant éventuellement substitué par un groupe hydroxy ou C₁₋₃ alcoxy ; W représente soit un atome d'hydrogène, soit un C₁₋₃ alkyle éventuellement substitué par un phényle, soit un phényle ;
K représente un groupe phényle ou pyridinyle, le groupe K étant éventuellement substitué par un ou plusieurs groupes choisis parmi un atome d'halogène, un groupe hydroxy, un C₁₋₃ alkyle, un C₁₋₃ alcoxy, un trifluorométhyle, un trifluorométhoxy, un -O-CHF₂ ;
à la condition qu'au moins un groupe R₄ ou R₅ représente un groupe Z ;
Z représente un groupe CN, SO₂NR₆R₇, ou un groupe hétéroaromatique ; ledit groupe hétéroaromatique étant éventuellement substitué par un groupe R₈ ; R₈ représentant soit un C₁₋₄ alkyle lui-même éventuellement substitué par un CN, un phényle ou un phénoxy ; soit un phényle ; les dits groupes phényle et phénoxy étant éventuellement substitués par 1 à 3 groupes choisis parmi un atome d'halogène, un C₁₋₃ alkyle, un C₁₋₃ alcoxy, un trifluorométhyle ;
R₆ et R₇ représentent, indépendamment l'un de l'autre, soit un atome d'hydrogène, soit un groupe C₁₋₆ alkyle éventuellement substitué par un C₃₋₇ cycloalkyle, un C₃₋₇ cycloalkènyle, C₁₋₃ alcoxy, un phényle, un naphtalènyle, un morpholinyle ou un pyridinyle ; soit un C₃₋₇ cycloalkyle, C₁₋₆ alcoxy, un phényle ou un indanyle ; les dits groupes C₃₋₇ cycloalkyle, C₃₋₇ cycloalkènyle, phényle, naphtalènyle, morpholinyle, pyridinyle et indanyle étant éventuellement substitués par un ou deux groupes choisis parmi un C₁₋₃ alkyle, un hydroxy, un C₁₋₃ alcoxy, un phényle ou un atome d'halogène ; ou
R₆ et R₇, avec l'atome d'azote qui les porte, forment un cycle aziridine, azétidine, pyrrolidine, pipéridine, morpholine ou benzopipéridine.

Parmi les composés de formule générale (I), un premier sous-groupe de composés est constitué par les composés pour lesquels :
R₁ représente un C₁₋₆ alkyle ou un phényle éventuellement substitué par 1 à 3 atomes d'halogène ; et/ou
R₂ et R'₂ représentent, indépendamment l'un de l'autre, un atome d'hydrogène ou un hydroxy ; et/ou
R₃ représente un C₁₋₆ alkyle ; et/ou
R₄ et R₅ représentent, indépendamment l'un de l'autre un atome d'hydrogène, un C₁₋₇ alkyle, un trifluorométhyle, un groupe L ou un groupe Z ; 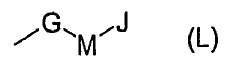
G représente un C₁₋₇alkyle ou une liaison simple ; et/ou
M représente un phényle éventuellement substitué par un ou plusieurs atomes d'halogène ; et/ou
J représente un atome d'hydrogène ou un groupe -Y-K ; et/ou
Y représente une liaison simple, un atome d'oxygène, -O-C₁₋₄ alkylène- ; et/ou
K représente un groupe phényle éventuellement substitué par un ou plusieurs groupes choisis parmi un atome d'halogène, un C₁₋₃ alkyle, un trifluorométhyle ; à la condition qu'au moins un groupe R₄ ou R₅ représente un groupe Z ; et/ou
Z représente un groupe CN, SO₂NR₆R₇, ou un groupe hétéroaromatique ; ledit groupe hétéroaromatique étant éventuellement substitué par un groupe R₈ ; R₈ représentant soit un C₁₋₄ alkyle lui-même éventuellement substitué par un phényle ; soit un phényle ; et/ou
R₆ et R₇ représentent, indépendamment l'un de l'autre, soit un atome d'hydrogène, soit un groupe C₁₋₆ alkyle éventuellement substitué par un phényle ou par un naphtalènyle ; soit un phényle ou un indanyle ; les dits groupes phényle étant éventuellement substitués par un ou deux groupes choisis parmi un C₁₋₃ alcoxy, un phényle ou un atome d'halogène ; ou
R₆ et R₇, avec l'atome d'azote qui les porte, forment un cycle benzopipéridine.

Les composés pour lesquels à la fois R₁, R₂, R'₂, R₃, R₄, R₅, G, M, J, Y, K, Z, R₆, R₇ et R₈ sont tels que définis dans le premier sous-groupe de composés ci-dessus, forme un second sous-groupe.

Parmi les composés de formule générale (I) et des sous-groupes ci-dessus, un troisième sous-groupe de composés est constitué par les composés pour lesquels :
R₁ représente un C₁₋₄ alkyle, de préférence un isopropyle ou un ter-butyle, ou un phényle substitué par deux atomes de fluor ; et/ou
R₂ et R'₂ représentent, indépendamment l'un de l'autre, un atome d'hydrogène ou un hydroxy ; et/ou
R₃ représente un C₁₋₄ alkyle, de préférence un méthyle, éthyle ou propyle.

Dans le cadre de la présente invention, on entend par :
- C_{t-z} où t et z peuvent prendre les valeurs de 1 à 7, une chaîne carbonée pouvant avoir de t à z atomes de carbone, par exemple C₁₋₃ une chaîne carbonée qui peut avoir de 1 à 3 atomes de carbone, C₃₋₆ une chaîne carbonée qui peut avoir de 3 à 6 atomes de carbone, ......
- alkyle, un groupe aliphatique saturé, linéaire ou ramifié; par exemple, un groupe C₁₋₆ alkyle représente une chaîne carbonée de 1 à 6 atomes de carbone, linéaire ou ramifiée, plus particulièrement un méthyle, éthyle, propyle, 1-méthyléthyle, butyle, isobutyle, sec-butyle, ter-butyle, .....de préférence un méthyle, éthyle, propyle ou 1-méthyléthyle ;
- alkylène, un groupe alkyle divalent saturé, linéaire ou ramifié, par exemple un groupe C₁₋₃-alkylène représente une chaîne carbonée divalente de 1 à 3 atomes de carbone, linéaire ou ramifiée, plus particulièrement un méthylène, éthylène, isopropylène, propylène ;
- cycloalkyle, un groupe alkyle cyclique, par exemple, un groupe C₃₋₇ cycloalkyle représente une chaîne carbonée cyclique de 3 à 7-atomes de carbone, plus particulièrement un cyclopropyle, cyclobutyle, cyclopentyle, cyclohexyle, cycloheptyle, de préférence un cyclopentyle ou cyclohexyle ;
- cycloalkènyle, un groupe alkyle cyclique mono- ou poly-insaturé, par exemple, un groupe C₃₋₇ cycloalkènyle représente une chaîne carbonée cyclique mono-ou poly-insaturée de 3 à 7 atomes de carbone, plus particulièrement un cyclopropényle, cyclobutènyle, cyclopentènyle, cyclohexènyle, cycloheptènyle, de préférence un cyclopentènyle ou cyclohexènyle ;
- thioalkyle, un groupe S-alkyle à chaîne aliphatique saturée, linéaire ou ramifiée ;
- alcoxy, un groupe O-alkyle à chaîne aliphatique saturée, linéaire ou ramifiée ;
- atome d'halogène, un fluor, un chlore, un brome ou un iode ;
- « R₂ et R_{2'} forment ensemble un groupe oxo », le groupe tel que : 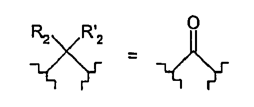
- groupe hétéroaromatique: un groupe aromatique cyclique comprenant entre 1 et 10 atomes de carbone et entre 1 et 4 hétéroatomes, tels que l'azote, l'oxygène ou le soufre. A titre d'exemples de groupes hétéroaromatiques, on peut citer les groupes oxazolyle, oxadiazolyle, tétrazolyle, benzoxazolyle...

Les composés de formule générale (I) peuvent comporter un ou plusieurs carbones asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention. Lorsque le carbone portant R₂ et R'₂ et/ou le carbone portant R₃ sont asymétriques, on préfère les composés de formule générale (I) pour lesquels le carbone portant R₂ et R'₂ est de configuration (S) et/ou le carbone portant R₃ est de configuration (S).
Les composés de formule (I) peuvent exister à l'état de bases ou de sels d'addition à des acides. De tels sels d'addition font partie de l'invention.
Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule générale (I) peuvent se trouver sous forme d'hydrates ou de solvats, à savoir sous forme d'associations ou de combinaisons avec une ou plusieurs molécules d'eau ou avec un solvant. De tels hydrates et solvats font également partie de l'invention.
La présente invention a pour second objet des procédés de préparation des composés de formule (I).

Ainsi, ces composés peuvent être préparés par des procédés, illustrés dans les schémas qui suivent, dont les conditions opératoires sont classiques pour l'homme du métier.
On entend par groupe protecteur, un groupement permettant d'empêcher la réactivité d'une fonction ou position, lors d'une réaction chimique pouvant l'affecter, et qui restitue la molécule après clivage selon des méthodes connues de l'homme du métier. Des exemples de groupes protecteurs ainsi que les méthodes de protection et déprotection sont données, entre autres, dans Protective groups in Organic Synthesis, Greene et coll., 2nd Ed. (John Wiley & Sons, Inc., New York).
Les significations de R₁, R₂, R'₂, R₃, R₄, R₅, G, M, J, Y, K, Z, R₆, R₇ et R₈ dans les composés de formule (II) à (XXI) ci-après sont telles que définies pour les composés de formule (I), à moins qu'une autre définition ne soit précisée.

Selon le schéma 1 ci-après, le composé de formule (I) peut être obtenu par couplage peptidique du 2-aminothiazole de formule (III) avec l'acylaminoacide de formule (II) selon des conditions connues de l'homme du métier, par exemple en présence d'héxafluorophosphate de benzotriazol-1-yloxy-tris(pyrrolidino) phosphonium (PyBOP) ou d'héxafluorophosphate de benzotriazol-1-yloxy-tris(diméthylamino)phosphonium (BOP) et de N-éthylmorpholine ou N-méthylmorpholine dans un solvant inerte tel que le N,N-diméthylformamide, l'acétonitrile ou le dichlorométhane à une température pouvant aller de 0°C à la température ambiante.

Le composé de formule (II) peut être obtenu par couplage peptidique du composé de formule (IV) avec l'acide protégé de formule (V), dans laquelle Pg représente un groupe protecteur, par exemple un benzyle, selon des méthodes connues de l'homme du métier telles que décrites ci-dessus.
Le composé ainsi obtenu est ensuite déprotégé. Dans le cas où la protection est un benzyle, le composé est préalablement hydrogéné en présence de palladium sur charbon dans de l'éthanol absolu à pression atmosphérique d'hydrogène, à température ambiante, pour donner le composé de formule (II).

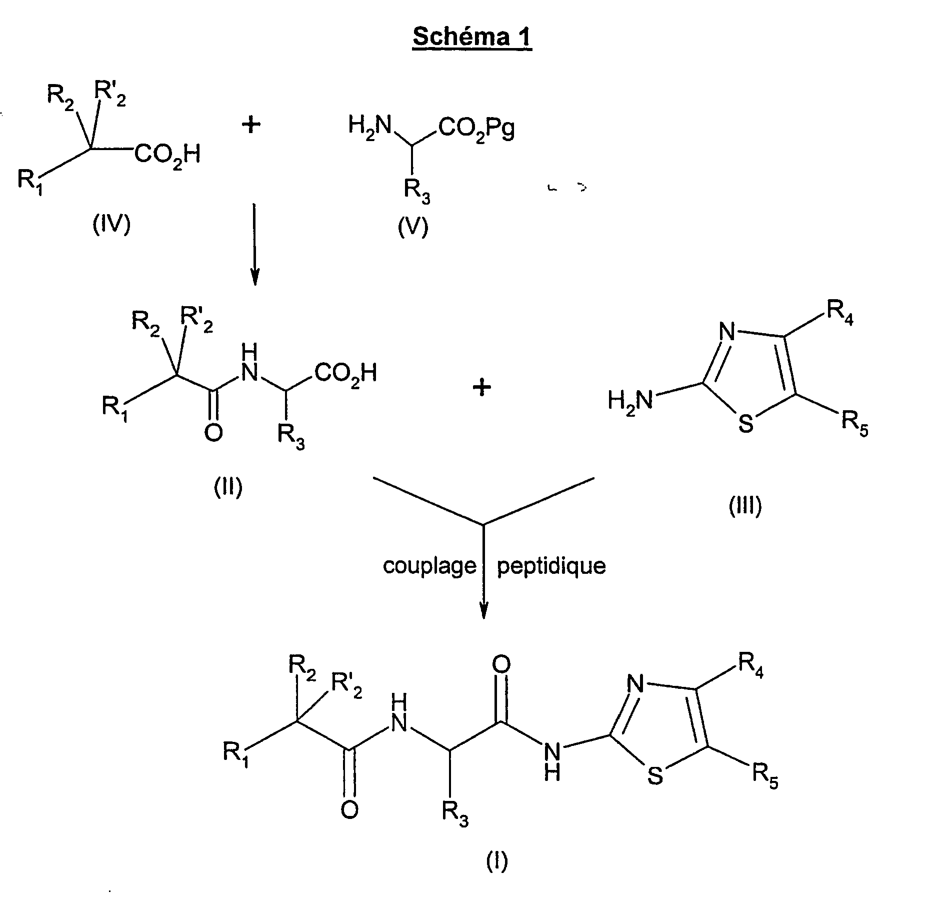

Alternativement, le composé de formule (I) peut être préparé selon le schéma 2 ci-après.

Selon le schéma 2, le composé de formule (I) peut être obtenu par couplage peptidique du composé de formule (IV) avec l'amine de formule (VI), selon des méthodes connues de l'homme du métier, comme par exemple en présence d'hydrate d'hydroxybenzotriazole (HOBt) et de chlorhydrate de 1-éthyl-3-(3-diméthylamino-propyl)carbodiimide (EDAC, HCl).

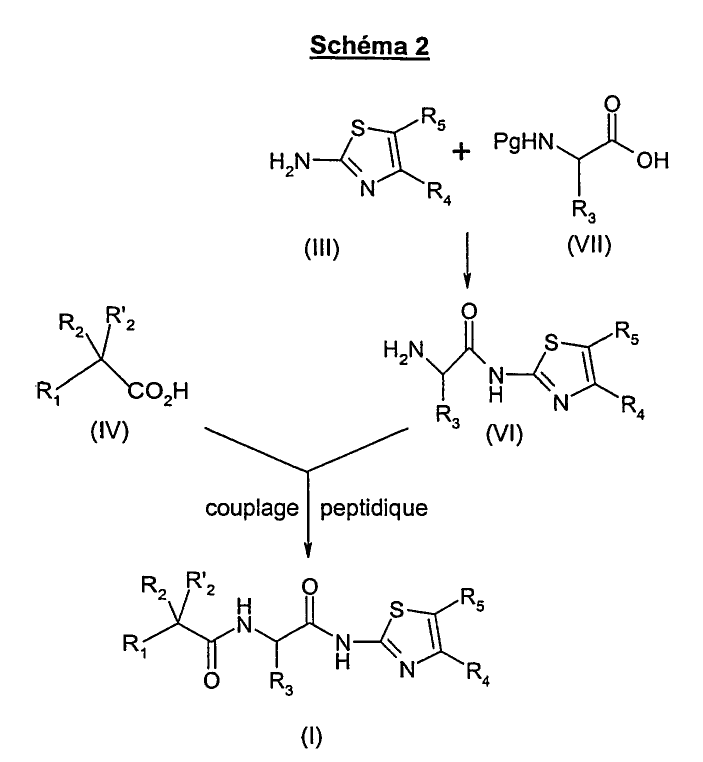

Le composé de formule (VI) peut être obtenu par couplage peptidique du 2-aminothiazole de formule (III) avec l'amine protégée de formule (VII) dans laquelle Pg représente un groupe protecteur, par exemple un *N-tert-*butoxycarbonyl (Boc), selon des méthodes connues de l'homme du métier telles que décrites ci-dessus. Le composé ainsi obtenu est ensuite déprotégé. Dans le cas où la protection est un Boc, la déprotection se fait par hydrolyse acide, en présence d'acide chlorhydrique gazeux dissous dans un solvant anhydre ou d'acide trifluoroacétique, pour donner le composé de formule (VI). Les composés de formule (I) dans laquelle R₂ et R'₂ forme un groupe oxo, peuvent être obtenu par oxydation d'un hydroxy du composé de formule (I) dans laquelle R₂ ou R'₂ représente un groupe hydroxy. La réaction peut être réalisée selon les conditions connues de l'homme du métier, par exemple avec le réactif de Dess Martin. Ces composés peuvent également être obtenus par couplage direct d'un cétoacide de formule (IV), dans laquelle R₂ et R'₂ forment ensemble un groupe oxo, avec une amine de formule (VI) selon les conditions connues de l'homme du métier. Les méthodes de préparation de tels cétoacides sont connues de l'homme du métier.

Les composés de formule (III) dans laquelle R₄ ou R₅ = Z, où Z représente un groupe CN ou un groupe de type hétéroaromatique, peuvent être préparés suivant les schémas 3 à 6 ci-après.

Les composés de formule générale (IIIa) et (IIIb), c'est-à-dire les composés de formule générale (III) dans laquelle R₄ ou R₅ représente un groupe oxadiazole peuvent être obtenus selon les méthodes illustrées par les schémas 3 et 4 ci-après.

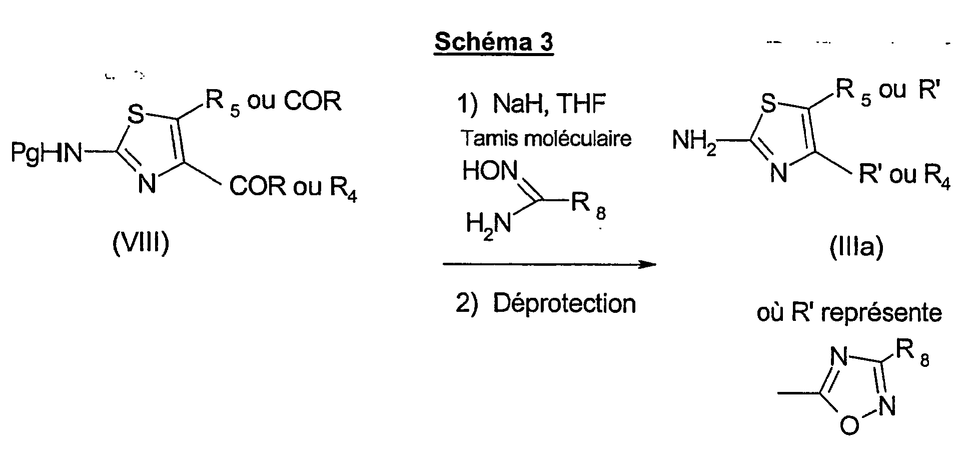

Selon le schéma 3 ci-dessus, le composé de formule (IIIa) peut être obtenu par réaction du composé de formule (VIII), dans laquelle R représente un groupe C₁₋₆ alcoxy et Pg représente un groupe protecteur tel qu'un groupe tert-butoxycarbonyle (Boc), avec une amidoxime de formule H₂NC(=NOH)R₈ dans du tétrahydrofurane anhydre à reflux en présence d'hydrure de sodium et de tamis moléculaires 4Å en poudre. Le composé ainsi obtenu est ensuite déprotégé selon les conditions connues de l'homme de métier.

Selon le schéma 4 ci-dessous, le composé de formule (X) est obtenu par couplage peptidique du composé de formule (IX), dans laquelle Pg représente un groupe protecteur tel qu'un Boc, avec de l'ammoniaque, en présence par exemple d'HOBt et de (EDAC,HCl). Le composé de formule (X) peut ensuite être mis en réaction avec de l'anhydride trifluoroacétique (TFAA) en présence d'une base par exemple la triéthylamine pour donner le composé de formule (XI).

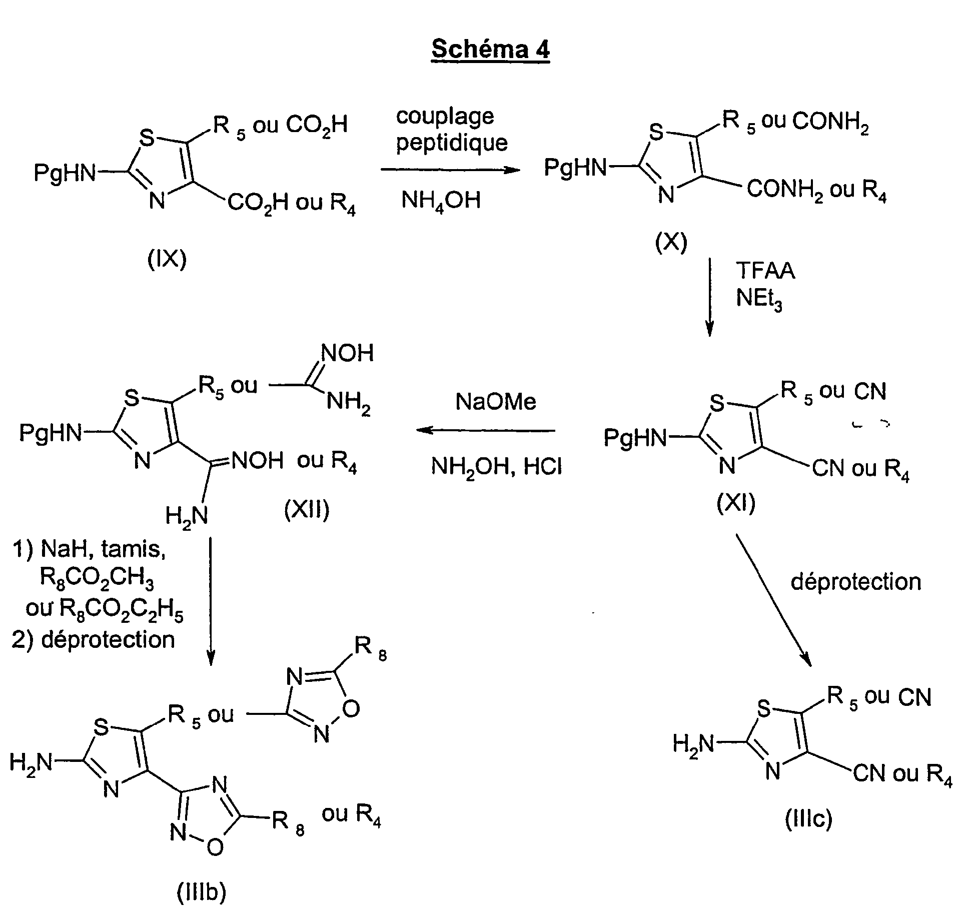

Le composé de formule (XII) peut être obtenu par addition d'hydroxylamine, HCl sur le composé de formule (XI), en présence d'une base, par exemple le méthylate de sodium, selon une adaptation du procédé décrit par Moloney et coll. (J. Chem. Soc. Perkin Trans I, 1999, p. 2725). Le composé de formule (XII) peut ensuite être cyclisé en présence d'une base telle que l'hydrure de sodium et d'un ester de formule RₐCO₂CH₃ ou R₈CO₂C₂H₅. Le composé ainsi obtenu est déprotégé selon les conditions connues de l'homme de métier pour obtenir le composé de formule générale (IIIb).

La méthode de préparation décrite dans le schéma 4 peut également permettre de préparer les composés de formule générale (IIIc), c'est-à-dire les composés de formule générale (III) dans laquelle R₄ ou R₅ représente un groupe -CN, par déprotection des composés de formule (XI) selon les conditions connues de l'homme de métier.

Les composés de formule générale (IIId), c'est-à-dire les composés de formule générale (III) dans laquelle R₄ ou R₅ représente un groupe benzoxazole peuvent être obtenus selon la méthode illustrée par le schéma 5 ci-après.

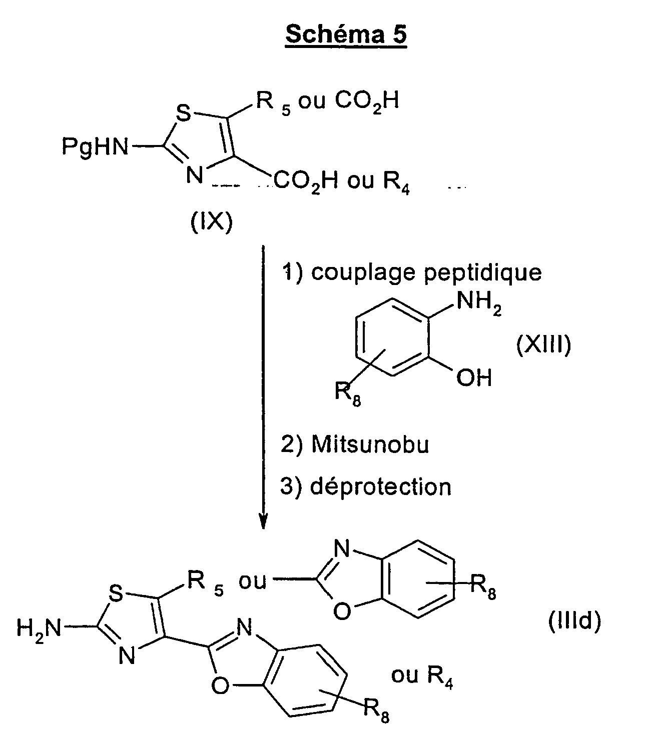

Selon le schéma 5, le composé de formule (IIId) peut être obtenu par couplage peptidique du composé de formule (IX) tel que défini ci-dessus et d'un composé de formule (XIII), selon les conditions connues de l'homme de métier, par exemple en présence d'héxafluorophosphate de benzotriazol-1-yloxy-tris(pyrrolidino)phosphonium (PyBOP) ou d'héxafluoro-phosphate de benzotriazol-1-yloxy-tris(diméthylamino)phosphonium (BOP) et de N-éthylmorpholine ou N-méthylmorpholine dans un solvant inerte tel que le N,N-diméthylformamide à une température pouvant aller de 0°C à la température ambiante.
Le composé ainsi obtenu peut ensuite être cyclisé par une réaction de Mitsunobu selon une adaptation du procédé décrit par Wang et coll. (Tetrahedron Letters, 1997, p. 6529) puis déprotégé par les méthodes connues de l'homme de métier pour donner le composé de formule générale (IIId).

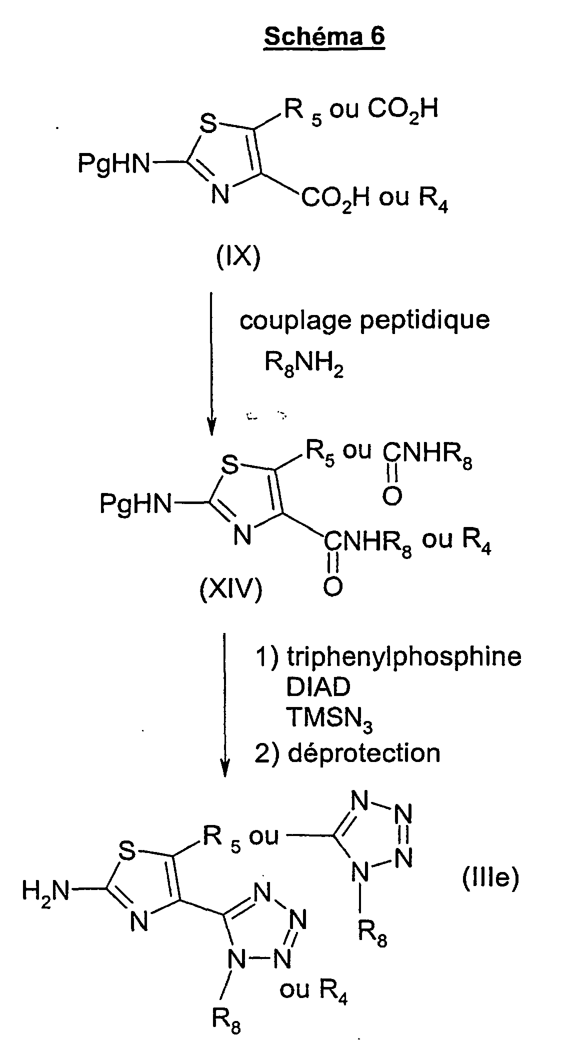

Les composés de formule générale (IIIe), c'est-à-dire les composés de formule générale (III) dans laquelle R₄ ou R₅ représente un groupe tétrazole peuvent être obtenus selon la méthode illustrée par le schéma 6 ci-dessus.
Selon le schéma 6, le composé de formule (XIV) peut être obtenu par couplage peptidique du composé de formule (IX) tel que défini ci-dessus et d'une amine primaire de formule RₐNH₂, selon les conditions connues de l'homme de métier par exemple en présence de HOBt et de (EDAC, HCl). Le composé (XIV) ainsi obtenu est soumis à une réaction de Mitsunobu selon les conditions connues de l'homme de métier par exemple en présence de triphénylphosphine, de düsopropylazodicarboxylate (DIAD) et de azidotriméthylsilane (TMSN₃), selon une adaptation du procédé décrit par De Lombaert et coll. dans J. Med. Chem. 2000, p. 488. Le composé obtenu est ensuite déprotégé par les méthodes connues de l'homme de métier pour donner le composé de formule générale (IIIe).

Les composés de formule générale (III) dans laquelle R₄ ou R₅ représente un groupe oxazole peuvent être obtenus à partir de l'aldéhyde correspondant, lui même préparé à partir de l'ester de formule (VIII), selon des procédures connues de l'homme du métier, par exemple avec de l'isocyanide p-toluenesulphonylmethyle en présence d'une base, telle que le carbonate de potassium ou du méthylate de sodium, selon une adaptation de la méthode décrite par van Leusen et coll. (Tetrahedron Letters, 1972, p. 2369).

Le composé de formule (VIII), tel que défini ci-dessus, peut être obtenu par protection d'un composé de formule (IIIo), 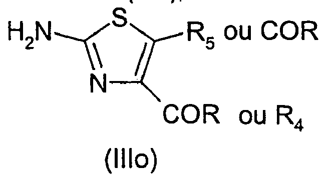 par exemple quand Pg représente un groupe Boc par action de di-tert-butyldicarbonate dans du tétrahydrofurane anhydre en présence de diméthylaminopyridine à température ambiante.
Le composé de formule (IX), tel que défini ci-dessus, peut être obtenu par hydrolyse de la fonction ester du composé de formule (VIII) correspondant selon des conditions connues de l'homme de métier, par exemple avec de l'hydroxyde de lithium dans un mélange tétrahydrofurane/eau 7 :3 (v/v) à une température de 60°C.

Les composés de formule (IIIo) peuvent être préparés selon les méthodes illustrées par les schémas 7 et 8 ci-après.
Le composé de formule (IIIo) dans laquelle R₄=-C(O)R, R représentant un groupe C₁₋₆ alcoxy, peut être obtenu selon le schéma 7 ci-après.

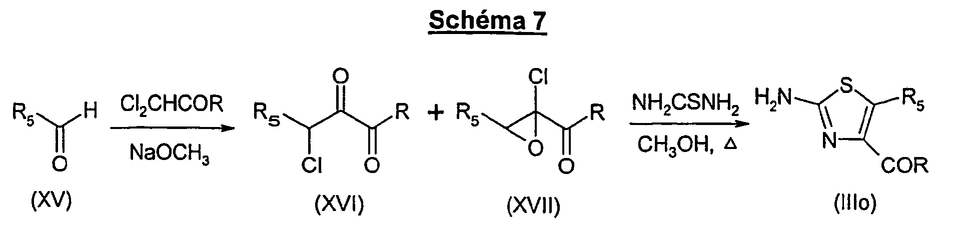

Selon le schéma 7, le composé de formule (IIIo) peut être obtenu par réaction d'un aldéhyde de formule (XV) avec le dichlorométhylacétate de formule Cl₂CHCOR, dans laquelle R représente un C₁₋₆ alcoxy, et par exemple le méthylate ou l'éthylate de sodium à 0°C selon une adaptation du procédé décrit par Takeda (Bull. Chem. Soc. JP, 1970, p. 2997). Le mélange de produits (XVI) et (XVII) obtenu est traité par la thiourée en présence par exemple de méthanol ou d'éthanol à reflux pendant 4 ou 8 heures pour donner le composé de formule (IIIo).
Le composé de formule (IIIo) dans laquelle R₅=-C(O)R, R représentant un groupe C₁₋₆ alcoxy, peut être obtenu selon le schéma 8.

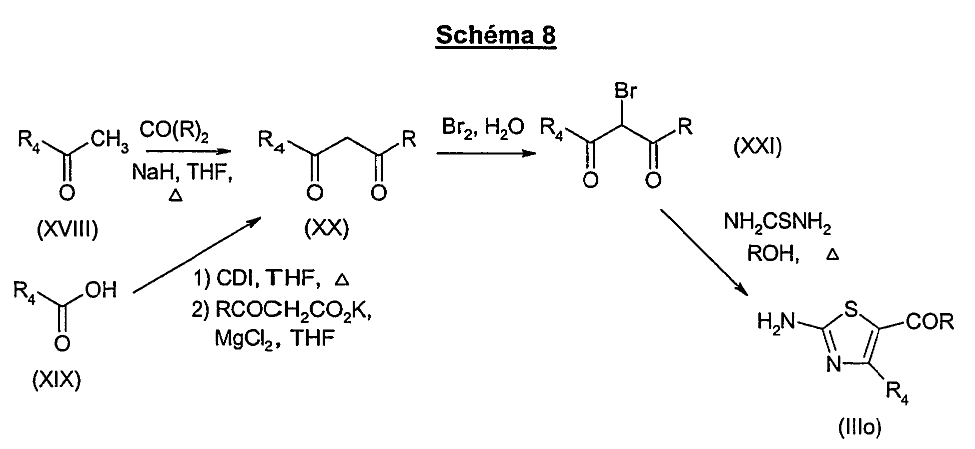

Selon le schéma 8, le composé de formule (IIIo), peut être obtenu par bromation d'un β-céto-ester de formule (XX), dans laquelle R représente un C₁₋₆ alcoxy, suivie d'une réaction avec la thiourée sur le composé (XXI) ainsi obtenu, selon une adaptation du procédé décrit par A. Barton et coll. (J.C.S Perkin I, 1982, p.159).
Le β-céto-ester de formule (XX) peut être obtenu par réaction d'une cétone de formule (XVIII) avec un dialkylcarbonate de formule CO(R)₂ dans laquelle R représente un C₁₋₆ alcoxy, selon une adaptation du procédé décrit par L. Crombie et coll. (J.C.S Perkin Trans 1, 1987, p.323). Le β-céto-ester de formule (XX) peut également être obtenu par réaction d'un acide de formule (XIX) activé par le carbonyldiimidazole (CDI) avec un malonate de formule RCOCH2CO2K, dans laquelle R représente un C₁₋₆ alcoxy, selon une adaptation du procédé décrit par exemple par D.W. Brooks et coll. (Angew. Chem. Int. Ed., 1979, p.72).
Dans le cas où R₄ représente un atome d'hydrogène, la préparation du composé de formule (XX) se fait selon une adaptation du procédé décrit par exemple par R. Zhao et coll. dans Tetrahedron Letters, 2001, p.2101.

Les composés de formule (III) dans laquelle R₄ ou R₅ = Z, où Z représente un groupe SO₂NR₆R₇ peuvent être préparés à partir des chlorures de sulfonyle correspondants disponibles dans le commerce ou à partir de composés décrits dans la littérature (par exemple par R. P. Fatheree et coll. dans WO 99/26932A1, par I. T. Barnish et coll. dans J. Med. Chem., 1980, p.117 et par R. Fischer et coll. dans WO 01/047904A1), ou peuvent être préparés par des méthodes qui y sont décrites ou qui sont connues de l'homme du métier.

Dans les schémas 1 à 8, les composés de départ et les réactifs, notamment les composés de formule (III), (IV), (V), (VII), (VIII), (IX), (XIII), (XV), (XVIII), (XIX), CO(R)₂, RC(O)CH₂CO₂K, Cl₂CHCOR, R₈NH₂, R₈CO₂CH₃, R₈CO₂C₂H₅, H₂NC(=NOH)R₈ quand leur mode de préparation n'est pas décrit, sont disponibles dans le commerce ou décrits dans la littérature, ou peuvent être préparés par des méthodes qui y sont décrites ou qui sont connues de l'homme du métier.
Par exemple, les composés de formule (IV) où R₂ ou R'₂ représente un hydroxy peuvent être préparés par addition de cyanure de triméthylsilyle sur un aldéhyde selon une adaptation du procédé décrit par D.A. Evans et coll. (J. C. S., Chem. Comm. 1973, p.55) ou par action du nitrite de sodium sur un alpha aminoacide selon une adaptation du procédé décrit par I. Shinn et coll. (J. Org. Chem., 2000, p.7667).
Par exemple, l'amidoxime de formule H₂NC(=NOH)R₈ peut être obtenue selon des procédés connus de l'homme de métier, par exemple selon la méthode décrite par Moloney et coll. dans J. Chem. Soc. Perkin Trans 1, 1999, p.2725.

Lorsqu'une fonction d'un composé est réactive, par exemple lorsque R₁ comporte un hydroxy, elle peut nécessiter une protection préalable avant réaction. L'homme du métier pourra déterminer aisément la nécessité d'une protection préalable.

Les exemples suivants décrivent la préparation de certains composés conformes à l'invention. Ces exemples ne sont pas limitatifs et ne font qu'illustrer l'invention.
Les numéros des composés exemplifiés renvoient à ceux donnés dans le tableau ci-après. Les micro-analyses élémentaires et les analyses RMN, IR ou LC-MS (chromatographie liquide couplée à la spectrométrie de masse) confirment les structures des composés obtenus.

### Exemple 1 :

### 2-{2-(S)-[2-(S)-hydroxy-(3-méthyl)butyrylaminolpentanoyl}amino-5-(1-méthyléthyl-4-(3-phényl-1,2,4-oxadiazol-5-yl)thiazole (composé n°8)

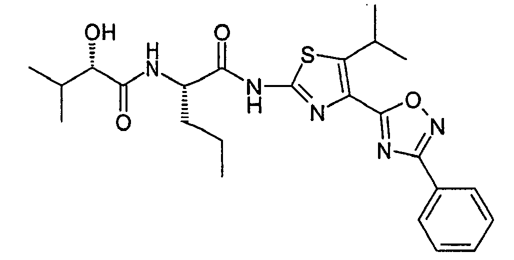

### Exemple 1.1 :

### 2-amino-5-(1-méthyléthyl)thiazole 4-carboxylate de méthyle

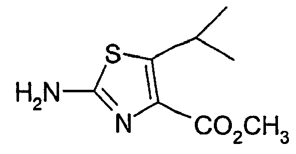

A 14,4 g d'isobutyraldéhyde en solution dans 400 ml de diéthyléther, on additionne, à 0°C, 24,6 g de dichloroacétate de méthyle, puis goutte à goutte 400 ml d'une solution de méthylate de sodium (0.5 M) dans du méthanol. Après 1 h à 0°C, on additionne 100 ml d'une solution aqueuse saturée en chlorure de sodium et on extrait à l'éther. On sèche la phase organique sur sulfate de sodium anhydre. On évapore uniquement l'éther en conservant le méthanol, on additionne 8 g de thiourée et on chauffe à reflux pendant 6 heures. On évapore le milieu réactionnel à sec, on le reprend à l'acétate d'éthyle et on le lave avec une solution aqueuse à 10% d'hydroxyde d'ammonium, puis avec une solution aqueuse saturée en chlorure de sodium. On sèche la phase organique sur sulfate de sodium anhydre, puis on la concentre. On reprend le résidu par 100 ml d'éther et on le filtre sur fritté. On obtient 18,6 g d'un solide blanc.
RMN 300 MHz (CDCl₃) δ ppm : 1,25 (d,6H) ; 3,35 (s,3H) ; 4,10 (m,1H) ; 5,50 (s,2H).

### Exemple 1.2 :

### 2-tert-butoxycarbonylamino-5-(1-méthyléthyl)thiazole 4-carboxylate de méthyle

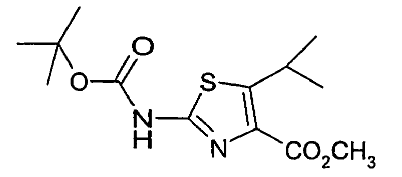

A 5,81 g de 2-amino-5-(1-méthyléthyl)thiazole 4-carboxylate de méthyle, obtenu à l'étape 1.1, en solution dans 300 ml de tétrahydrofurane, on additionne 6,96 g de di-tert-butyldicarbonate et 0,177 g de diméthylaminopyridine. On agite le mélange à température ambiante pendant 16 h. On évapore le milieu réactionnel. On reprend le résidu à l'acétate d'éthyle, et on le lave 2 fois avec une solution aqueuse 0,5 N d'acide chlorhydrique, 1 fois à l'eau, puis avec une solution aqueuse saturée en chlorure de sodium. On sèche la phase organique sur sulfate de sodium anhydre et on la concentre. On obtient 8,35 g du dérivé aminothiazole protégé sous forme d'un solide qu'on utilise tel quel sans purification.
LC/MS : MH⁺ = 401 (M - Boc)⁺ = 301

### Exemple 1.3:

### 2-amino-5-(1-méthyléthyl)-4-(3-phényl-1,2,4-oxadiazol-5-yl)thiazole

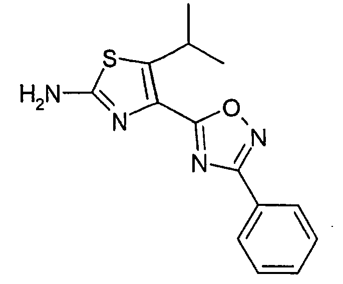

A 2,9 g de benzamidoxime, en solution dans 150 ml de tétrahydrofurane, on additionne à température ambiante lentement 0.96 g d'hydrure de sodium et 2 g de tamis moléculaires 4Å. On chauffe le mélange à 60° pendant 1 h30. On laisse revenir le mélange à température ambiante puis on ajoute 3.6 g de 2-tert-butoxycarbonylamino-5-(1-méthyléthyl)thiazole 4-carboxylate de méthyle, obtenu à l'étape 1.2, dans 30 ml de tétrahydrofurane. Le mélange réactionnel est chauffé à 60° pendant 12 heures. La réaction est stoppée par ajout d'eau. On filtre les tamis moléculaires puis on concentre la solution. Le produit est repris dans l'acétate d'éthyle. Après évaporation à sec, on obtient 3,4 g d'un solide blanc.
On place sous agitation 3,4 g du produit obtenu ci-dessus en solution dans 60 ml d'acide trifluoroacétique à température ambiante pendant 30 minutes, puis on l'évapore. On reprend le résidu à l'acétate d'éthyle, et on le lave 2 fois avec une solution aqueuse saturée en carbonate de sodium, puis avec une solution aqueuse saturée en chlorure de sodium. On sèche la phase organique sur sulfate de sodium anhydre, puis on l'évapore pour donner 1,8 g d'un solide blanc.
RMN 300 MHz (CDCl₃) δ ppm : 1,38 (d,6H) ; 4,21 (m,1H) ; 5,23 (s,2H) ; 7,50 (m,3H) ; 8,20 (m,2H).

### Exemple 1.4:

### 2-[2-(S)-pentanoylarnino]amino-5-(1-méthyléthyl)-4-(3-phényl-1,2,4-oxadiazol-5-yl)thiazole

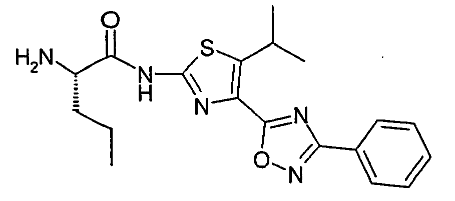

A 1,77 g de 2-amino-5-(1-méthyléthyl)-4-(3-phényl-1,2,4-oxadiazol-5-yl)thiazole, obtenu à l'étape 1.3, en solution dans 50 ml de diméthylformamide à 0°C, on additionne 0,75ml de N-méthylmorpholine, 3,5 g de PyBOP puis 1,5 g de (S)-Bocnorvaline. On laisse revenir le milieu réactionnel à température ambiante puis on l'agite pendant 16 h. Après évaporation, on reprend le résidu par de l'acétate d'éthyle et on le lave 2 fois avec une solution aqueuse saturée en bicarbonate de sodium, 2 fois à l'eau, 1 fois avec une solution aqueuse 1H d'hydrogénosulfate de potassium, puis avec une solution aqueuse saturée en chlorure de sodium. On sèche la phase organique sur sulfate de sodium anhydre, puis on la concentre. On chromatographie le résidu sur une colonne de gel de silice, en éluant avec un mélange de dichlorométhane et d'acétate d'éthyle 95:5 (v/v). On obtient 1,4 g d'un solide blanc.
On agite 1,4 g de produit obtenu ci-dessus en solution dans 60 ml d'acide trifluoroacétique à température ambiante pendant 30min, puis on l'évapore. On reprend le résidu à l'acétate d'éthyle, et on le lave 2 fois avec une solution aqueuse saturée en carbonate de sodium, puis avec une solution aqueuse saturée en chlorure de sodium. On sèche la phase organique sur sulfate de sodium anhydre, puis on l'évapore pour donner 1 g d'un solide blanc.
RMN 300 MHz (CDCl₃) δ ppm : 0,98 (t,3H) ; 1,45 (d,6H) ; 1,62 (m,2H) ; 1,92 (m,2H) ; 3,64 (m,1H) ; 4,25 (m,1H) ; 7,50 (m,3H) ; 8,20 (m,2H).

### Exemple 1.5 :

### 2-{2-(S)-[2-(S)-hydroxy-(3-méthyl)butyrylamino]pentanoyl}amino-5-(1-méthyléthyl-4-(3-phényl-1,2,4-oxadᵢazol-5-yl)thiazole

A 0,2 g de 2-[2-(S)-pentanoylamino]amino-5-(1-méthyléthyl)-4-(3-phényl-1,2,4-oxadiazol-5-yl)thiazole, obtenu à l'étape 1.4, en solution dans 10 ml de diméthylformamide à 0°C, on additionne 0.06 ml de N-méthylmorpholine, 0,30 g de PyBOP puis 0,06 g d'acide α-hydroxyhydrovalérique. On laisse revenir la réaction à température ambiante et on l'agite pendant 18 h. On évapore le milieu réactionnel. On reprend le résidu à l'acétate d'éthyle et on le lave 2 fois avec une solution aqueuse saturée en bicarbonate de sodium, 2 fois à l'eau, 1 fois avec une solution aqueuse 1 M d'hydrogénosulfate de potassium, puis avec une solution aqueuse saturée en chlorure de sodium. On sèche la phase organique sur sulfate de sodium anhydre puis on la concentre. On chromatographie le résidu sur une colonne de silice en éluant avec un mélange dichlorométhane/acétate d'éthyle 95:5 (v/v) pour obtenir 0,15 g d'un solide blanc.
LC/MS : MH⁺ =486
RMN : décrite dans le tableau ci-après. (composé n°8)

### Exemple 2 :

### 2-{2-(S)-[2-(S)-hydroxy-(3,3-diméthyl)butyrylamino]pentanoyl}amino-5-(2-benzyloxy)phénylthiazole 4-nitrile (composé n°22)

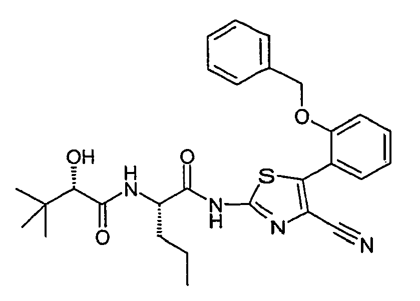

### Exemple 2.1 :

### 2-amino-5-(2-benzyloxy)phénylthiazole 4-carboxylate de méthyle

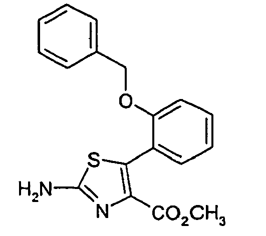

On procède de la même manière qu'à l'étape 1.1 de l'exemple 1 en remplaçant le l'isobutyraldéhyde par le 2-benzyloxybenzaldéhyde (42,25 g) en solution dans 400 ml de diéthyléther sur lequel on additionne à 0°C, 25,7 g de méthyldichloroacétate, puis goutte à goutte 400 ml d'une solution de méthylate de sodium (0.5 M) dans du méthanol. Après 1 h à 0°C, on additionne 100 ml d'une solution aqueuse saturée en chlorure de sodium et on extrait à l'éther. Après traitement avec la thiourée (10,66 g), on obtient 19 g d'un solide jaune.
LC/MS : MH⁺ = 341

### Exemple 2.2:

### 2-tert-butoxycarbonylamino-5-(2-benzyloxy)phénylthiazole 4-carboxylate de méthyle

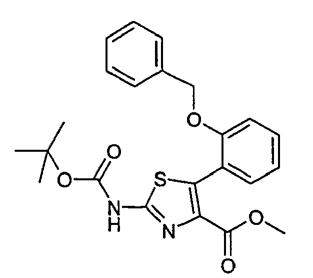

A 15,7 g de 2-amino-5-(2-benzyloxy)phénylthiazole 4-carboxylate de méthyle, obtenu à l'étape 2.1, en solution dans 300 ml de tétrahydrofurane, on additionne 11,5 g de di-tert-butyldicarbonate et 0,4 g de diméthylaminopyridine. On agite le mélange à température ambiante pendant 16 h. On évapore le milieu réactionnel. On reprend le résidu à l'acétate d'éthyle, et on le lave 2 fois avec une solution aqueuse 0,5N d'acide chlorhydrique, 1 fois à l'eau, puis avec une solution aqueuse saturée en chlorure de sodium. On sèche la phase organique sur sulfate de sodium anhydre et on la concentre. On obtient 20,5 g du dérivé aminothiazole protégé sous forme d'un solide qu'on utilise tel quel sans purification.
LC/MS : MH⁺ = 441 (M - Boc)⁺ = 341

### Exemple 2.3:

### 2-tert-butoxycarbonylamino-5-(2-benzyloxy)phénylthiazole 4-carboxylique acide

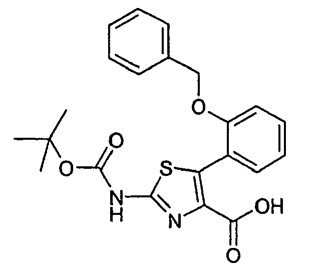

A 5,6 g de 2-tert-butoxycarbonylamino=5-(2-benzyloxy)phénylthiazole 4-carboxylate de méthyle, obtenu à l'étape 2.2, en solution dans 150 ml de tétrahydrofurane, on additionne à température ambiante une solution de 1,35 g d'hydroxyde de lithium dans 80 ml d'eau distillée. On chauffe le mélange réactionnel à reflux de tétrahydrofurane pendant 16 h, puis on le concentre. On reprend le résidu par de l'eau et on le lave 2 fois avec de l'acétate d'éthyle. On acidifie la phase aqueuse avec une solution 1 N d'acide chlorhydrique jusqu'à pH ~ 4, on la sature en chlorure de sodium et on l'extrait 2 fois avec de l'acétate d'éthyle. On sèche la phase organique sur sulfate de sodium anhydre, puis on la concentre.
LC/MS : MH⁺ = 427

### Exemple 2.4 :

### 2-tert-butoxycarbonylamino-5-(2-benzyloxy)phénylthiazole 4-amide

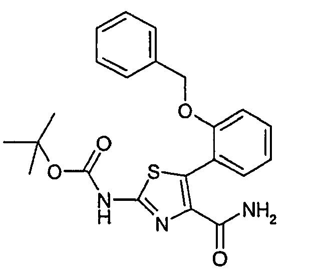

A 4,2 g de 2-tert-butoxycarbonylamino-5-(2-be nzyloxy)phénylthiazole 4-carboxylique acide obtenu à l'étape 2.3, en solution dans 150 ml de diméthoxyéthane à 0°, on additionne 1,3 g de N-méthylmorpholine puis 1,7 g d'isobutylchloroformate et 8 ml d'une solution d'hydroxyde d'ammonium à 25%. On agite le milieu réactionnel à température ambiante 16 h, puis on le concentre. On reprend le résidu à l'acétate d'éthyle et on le lave 2 fois avec une solution aqueuse 1 M d'hydrogénosulfate de potassium, 1 fois à l'eau, puis avec une solution aqueuse saturée en chlorure de sodium. On sèche la phase organique sur sulfate anhydre et on la concentre. On chromatographie le résidu sur une colonne de silice en éluant avec un mélange dichlorométhane/acétate d'éthyle 80/20 (v/v) pour obtenir 3,3 g d'un solide blanc.
LC/MS : MH⁺ = 426

### Exemple 2.5 :

### 2-tert-butoxycarbonylamino -5-(2-benzyloxy)phénylthiazole 4-nitrile

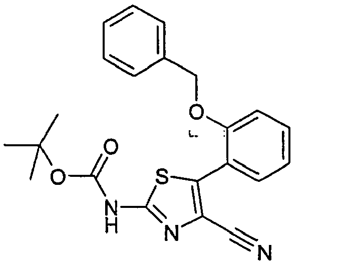

A 3,3 g 2-tert-butoxycarbonylamino-5-(2-benzyloxy)phénylthiazole 4-amide, obtenu à l'étape 2.4, à 0° dans 80 ml de dichlorométhane, on additionne 2,6 ml de triéthylamine et 1,7 ml d'anhydride trifluoroacétique. On agite le mélange à 0° pendant une heure puis à température ambiante pendant 12 heures, puis on le concentre. On extrait le résidu au dichlorométhane, et on le lave 2 fois avec une solution aqueuse 1 M d'hydrogénosulfate de potassium, puis avec une solution aqueuse saturée en chlorure de sodium. On sèche la phase organique sur sulfate de sodium anhydre, puis on la concentre. On obtient 1,1 g d'un solide jaune.
LC/MS : MH⁺ = 408
RMN 300 MHz (CDCl₃) δ ppm : 1,55 (s,9H) ; 5,22 (s,2H) ; 7,00-7,80 (m,9H).

### Exemple 2.6 :

### 2-{2-(S)-[2-(S)-hydroxy-(3,3-diméthyl)butyrylamino]pentanoyl}amino-5-(2-benzyloxy)phénylthiazole 4-nitrile

Le composé obtenu à l'étape 2.5 est ensuite déprotégé par de l'acide trifluoroacétique comme décrit à l'étape 1.3 de l'exemple 1, puis 2 couplages successifs sont réalisés selon les procédés décrits aux étapes 1.4 et 1.5 de l'exemple 1.
On obtient 0,1g de produit final.
RMN : décrite dans le tableau ci-après. (composé n°22)

### Exemple 3 :

### 2-{2-(S)-[2-(S)-hydroxy-(3,3-diméthyl)butyrylamino]pentanoyl}amino-5-(2-benzyloxy)phényl-4-(5-méthyl-1,2,4-oxadiazol-3-yl)thiazole (composé n°13)

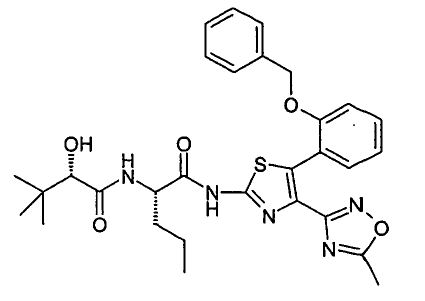

### Exemple 3.1:

### 2-tert-butoxycarbonylamino-5-(2-benzyloxy)phénylthiazole 4-amideoxime

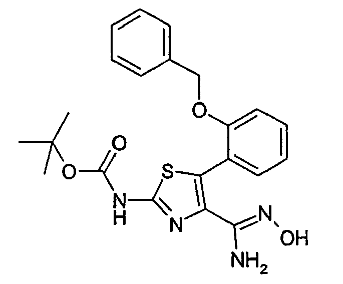

A 4 ml d'une solution de méthoxyde de sodium 0,5M dans le méthanol, on ajoute goutte à goutte 0,136 g d'hydroxylamine dans le méthanol. Il se forme un précipité blanc, le mélange réactionnel est agité une heure à température ambiante puis on y ajoute 0,8 g du composé 2-tert-butoxycarbonylamino-5-(2-benzyloxy)phénylthiazole 4-nitrile obtenu à l'étape 2.5 de l'exemple 2. Le mélange est chauffé pendant 15 heures puis on évapore le méthanol. On extrait le résidu dans l'acétate d'éthyle, et on le lave 2 fois avec une solution aqueuse saturée en chlorure de sodium. On sèche la phase organique sur sulfate de sodium anhydre, puis on la concentre. On obtient 0,67 g d'un solide.
LC/MS : MH⁺ = 441

### Exemple 3.2: 2-tert-butoxycarbonylamino-5-(2-benzyloxy)phényl-4-(5-méthyl-1,2,4-oxadiazol-3-yl)thiazole

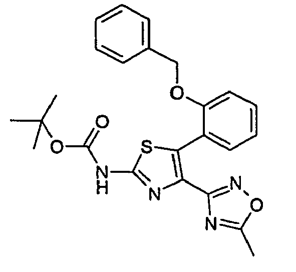

A 0,66 g de 2-tert-butoxycarbonylamino-5-(2-benzyloxy)phénylthiazole 4-amideoxime obtenu dans l'étape 3.1, en solution dans 30 ml de tétrahydrofurane, on additionne à température ambiante lentement 0.75 g d'hydrure de sodium et 0,5 g de tamis moléculaires 4Å. On chauffe le mélange à 60° pendant 1 h et 30 minutes, puis on ajoute 220 µl d'acétate d'éthyle et on continue à chauffer à 60°C pendant 12h. La réaction est stoppée par ajout d'eau. On filtre les tamis moléculaires puis on concentre la solution. Le produit est repris dans l'acétate d'éthyle. Après évaporation à sec, on obtient 0,58 g d'un solide blanc.
RMN 300 MHz (CDCl₃) δ ppm : 1,53 (s,9H) ; 2,48 (s,3H) ; 5,04 (s,2H) ; 6,90-7,42 (m,9H) ; 8,00 (s,1 H).

### Exemple 3.3: 2-amino-5-(2-benzyloxy)phényl-4-(5-méthyl-1,2,4-oxadiazol-3-yl)thiazole

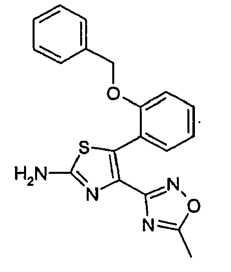

On agite 0,58 g de 2-tert-butoxycarbonylamino-5-(2-benzyloxy)phényl-4-(5-méthyl-1,2,4-oxadiazol-3-yl)thiazole, obtenu dans l'étape 3.2, en solution dans 30 ml d'acide trifluoroacétique à température ambiante pendant 30min, puis on l'évapore. On reprend le résidu à l'acétate d'éthyle, et on le lave 2 fois avec une solution aqueuse saturée en carbonate de sodium, puis avec une solution aqueuse saturée en chlorure de sodium. On sèche la phase organique sur sulfate de sodium anhydre, puis on l'évapore pour donner 0,46 g d'un solide blanc.
RMN 300 MHz (CDCl₃) δ ppm : 2,54 (s,3H) ; 5,07 (s,2H) ; 6,98-7,48 (m,9H).

### Exemple 3.4 :

### 2-{2-(S)-[2-(S)-hydroxy-(3,3-diméthyl)butyrylamino]pentanoyl}amino-5-(2-benzyloxy)phényl-4-(5-méthyl-1,2,4-oxadiazol-3-yl)thiazole

Deux couplages successifs sont réalisés selon les procédés décrits aux étapes 1.4 et 1.5 de l'exemple 1.
On obtient 0,270g de produit final.
RMN : décrite dans le tableau ci-après. (composé n°13)
LC/MS : MH⁺ = 578

### Exemple 4 :

### 2-{2-(S)-[2-(S)-hydroxy-(3,3-diméthyl)butyrylamino]pentanoyl}amino-5-(1-méthyléthyl)-4-(1,3-benzoxazol-2-yl)thiazole (composé n°34)

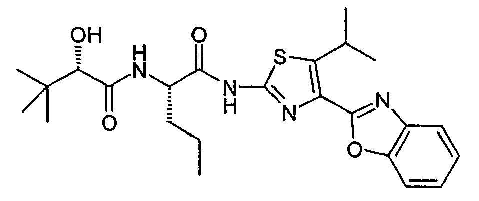

### Exemple 4.1 :

### 2-tert-butoxycarbonylamino-5-(1-méthyléthyl)thiazole 4-carboxylique acide

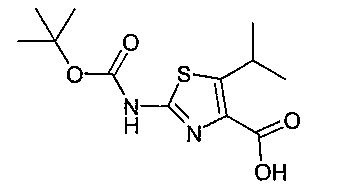

A partir du 2-tert-butoxycarbonylamino-5-(1-méthyléthyl)thiazole 4-carboxylate de méthyle, obtenu à l'étape 1.2 de l'exemple 1, on prépare l'acide correspondant (3 g) en utilisant le procédé décrit à l'étape 2.3 de l'exemple 2.
LC/MS : MH⁺ = 287
RMN 300 MHz (CDCl₃) δ ppm : 1,20 (d,6H) ; 1,50 (s,9H) ; 4,40 (m,1H).

### Exemple 4.2

### 2-tert-butoxycarbonylamino-5-(1-méthyléthyl)thiazole 4-(2-hydroxy)phényl carboxamide

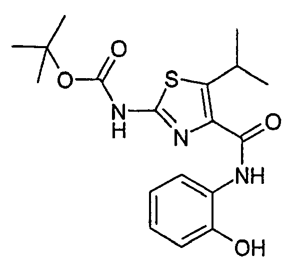

A une solution de 3 g de 2-tert-butoxycarbonylamino-5-(1-méthyléthyl)-thiazole 4-carboxylique acide, obtenu à l'étape 4.1, dans 75 ml de diméthylformamide à 0°, on additionne 1,2 g de N-méthylmorpholine, 6,11 g de PyBOP puis 1,3 g de 2-aminophénol. On laisse revenir le milieu réactionnel à température ambiante, on l'agite 16 h, puis on le concentre. On reprend le résidu par de l'acétate d'éthyle, on le lave 2 fois avec une solution aqueuse saturée en bicarbonate de sodium, 2 fois à l'eau, 1 fois avec une solution aqueuse 1 M d'hydrogénosulfate de potassium puis avec une solution aqueuse saturée en chlorure de sodium. On sèche la phase organique sur sulfate de sodium anhydre puis on la concentre. On chromatographie le résidu sur une colonne de gel de silice, en éluant avec un mélange d'éther de pétrole et d'acétate d'éthyle allant de 9:1 (7 :3) (v/v). On obtient 2,7 g d'une poudre orangée.
LC/MS : MH⁺ = 378.
RMN 300 MHz (CDCl₃) δ ppm : 1,35 (d,6H) ; 1,36 (s,9H) ; 4,33 (m,1H) ; 6,80-7,20 (m,4H) ; 7,78 (s,1H) ; 9,30 (s,1H) ; 9,52 (s,1H).

### Exemple 4.3

### 2-tert-butoxycarbonylamino-5-(1-méthyléthyl)-4-(1,3-benzoxazol-2-yl)thiazole

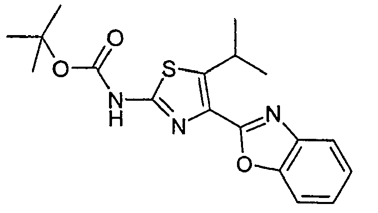

A une solution de 2,72 g de 2-tert-butoxycarbonylamino-5-(1-méthyléthyl)-thiazole 4-(2-hydroxy)phényl carboxamide, obtenu à l'étape 4.2, dans 80 ml de tétrahydrofurane à 0°, on additionne 2,1 g de triphénylphosphine puis goutte à goutte 1,86 g de DIAD dans 30 ml de tétrahydrofurane. On laisse lentement revenir le milieu réactionnel à température ambiante, on l'agite 16 h, puis on le concentre. On chromatographie le résidu sur une colonne de gel de silice, en éluant avec un mélange d'éther de pétrole et d'acétate d'éthyle allant de 9:1 (7 :3) (v/v). On obtient 1,2 g d'une poudre orangée.
LC/MS : MH⁺ = 360.
RMN 300 MHz (CDCl₃) δ ppm : 1,42 (d,6H) ; 1,50 (s,9H) ; 4,40 (m,1H) ; 7,30-7,80 (m,4H) ; 8,45 (s, 1H).

### Exemple 4.4 :2-{2-(S)-[2-(S)-hydroxy-(3,3-diméthyl)butyrylamino]-pentanoyl}amino-5-(1-méthyléthyl)-4-(1,3-benzoxazol-2-yl)thiazole

Le 2-tert-butoxycarbonylamino-5-(1-méthyléthyl)-4-(1,3-benzoxazol-2-yl)thiazole, obtenu à l'étape 4.3, est ensuite déprotégé par de l'acide trifluoroacétique comme décrite à l'étape 1.3 de l'exemple 1, puis 2 couplages successifs sont réalisés selon les procédés décrits aux étapes 1.4 et 1.5 de l'exemple 1.
On obtient 0,2g de produit final.
RMN : décrite dans le tableau ci-après. (composé n°34)
LC/MS : MH⁺ = 473

### Exemple 5 :

### 2-{2-(S)-[2-(S)-hydroxy-(3,3-diméthyl)butyrylamino]pentanoyl}amino-5-(1-benzyl-1H-tétrazole-5-yl)thiazole (composé n°51)

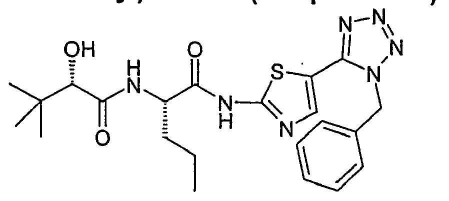

### Exemple 5.1: 2-tert-butoxycarbonylaminothiazole 5-carboxylate de méthyle

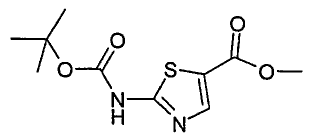

On dissout 17,42 g de 3-méthoxyacrylate de méthyle dans 200 ml d'un mélange 1/1 dioxane-1,4 /eau et on refroidit la solution à 0°C avant d'y ajouter 29,37 g de N-bromosuccinimide. Après 1 h à 0°C, on additionne 11,42 g de thiourée puis on chauffe à 80°C pendant 2 heures. Le mélange réactionnel est ensuite évaporé et le résidu repris dans l'acétate d'éthyle puis lavé deux fois avec une solution aqueuse d'hydroxyde de sodium à 10%, puis par une solution saturée en chlorure de sodium. La phase organique est séchée sur sulfate de magnésium anhydre puis concentrée. On obtient 19,17 g d'un solide beige.
LC/MS : MH⁺ = 159
L'amine de cet intermédiaire est ensuite protégée par un groupement Boc selon le procédé décrit à l'étape 1.2 de l'exemple 1. La masse de produit obtenue est de 30,7 g.
LC/MS : MH⁺ = 259
RMN 300 MHz (CDCl₃) δ ppm : 1,50 (s,9H) ; 3,80 (s,3H) ; 8,06 (s,1H).

### Exemple 5.2 : 2-tert-butoxycarbonylaminothiazole 5-benzylamide

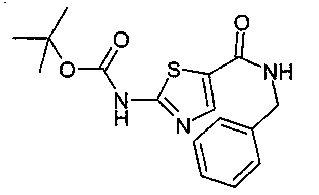

A partir du 2-tert-butoxycarbonylaminothiazole 5-carboxylate de méthyle obtenu à l'étape 5.1, on prépare l'acide correspondant comme décrit à l'étape 2.3 de l'exemple 2. A partir du 2-tert-butoxycarbonylaminothiazole 5-carboxylique acide ainsi obtenu, on prépare l'amide (4,35 g) comme décrit dans l'exemple 2.4.
LC/MS : MH⁺ = 334.

### Exemple 5.3 : 2-tert-butoxycarbonylamino-5-(1-benzyl-1H-tétrazole-5-yl)thiazole

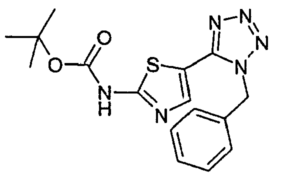

A une solution de 4,35 g du composé obtenu à l'étape 5.2 dans 200 ml de tétrahydrofurane à 0°, on additionne 10,3 g de triphénylphosphine puis goutte à goutte 7,92 g de DIAD dans 30 ml de tétrahydrofurane. Après 15 mn, on ajoute 4,5 g d'azidotriméthylsilane dilué dans 20 ml de tétrahydrofurane. On laisse revenir le milieu réactionnel à température ambiante, on l'agite 24 h, puis on le concentre. Le résidu est repris dans l'acétate d'éthyle, le précipité formé est filtré puis chromatographié sur une colonne de gel de silice, en éluant avec un mélange d'éther de pétrole et d'acétate d'éthyle 5 :5 (v/v). On obtient 4 g d'une huile orangée qui est rechromatographiée sur une colonne de gel de silice avec un mélange de dichlorométhane et d'acétate d'éthyle allant de 10:0 (8 :2) (v/v).
LC/MS : MH⁺ = 359.
RMN 300 MHz (CDCl₃) δ ppm : 1,50 (s,9H) ; 5,92 (s,2H) ; 7,18 (m,2H) ; 7,37 (m,3H) ; 7,98 (s,1H) ; 8,86 (s,1H).

### Exemple 5.4 :

### 2-{2-(S)-[2-(S)-hydroxy-(3,3-diméthyl)butyrylamino]pentanoyl}amino-5-(1-benzyl-1H-tétrazole-5-yl)thiazole

Le 2-tert-butoxycarbonylamino-5-(1-benzyl-1H-tétrazole-5-yl)thiazole, obtenu à l'étape 5.3, est ensuite déprotégé par de l'acide trifluoroacétique comme décrit à l'étape 1.3 de l'exemple 1, puis deux couplages successifs sont réalisés selon les procédés décrits aux étapes 1.4 et 1.5 de l'exemple 1.
On obtient 0,090g de produit final.
RMN : décrite dans le tableau ci-après. (composé n°51)
LC/MS : MH⁺ = 472.6

### Exemple 6 :

### 2-{2-(S)-[2-(3,5-difluorophényl)acétylamino]pentanoyl}amino-4-méthylthiazole 5-(N-2,3-dichlorobenzyl)sulfonamide (composé n°64)

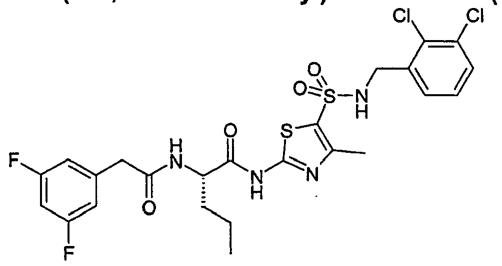

### Exemple 6.1 :

### 2-acétamido-4-méthylthiazole 5-(N-2,3-dichlorobenzyl)sulfonamide

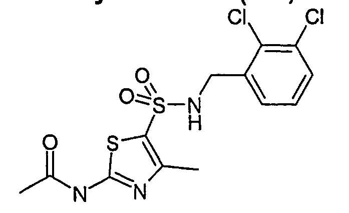

A 2,5 g de chlorure de 2-acétamido-4-méthylthiazole-5-sulfonyle commercial dans 60ml de dichlorométhane anhydre, à 0°C, on ajoute 2 équivalents (3.5g) de 2,3-dichlorobenzylamine. On laisse revenir à température ambiante. Après 5h environ, on extrait au dichlorométhane, on lave deux fois avec une solution d'hydrogénosulfate de sodium 1M puis deux fois avec une solution saturée de bicarbonate de sodium. La phase organique est lavée avec une solution saturée de chlorure de sodium puis séchée sur du sulfate de magnésium anhydre. Après filtration, évaporation et concrétisation dans un mélange dichlorométhane /pentane, on obtient 2.9 g (95%) d'une poudre blanche. RMN 300 MHz (DMSO) δ ppm : 2,16 (s,3H) ; 2,40 (s,3H) ; 4,21 (s,2H) ; 7,28-7,56 (m,3H).

### Exemple 6.2 : 2-amino-4-méthylthiazole 5-(N-2,3-dichlorobenzyl)-sulfonamide

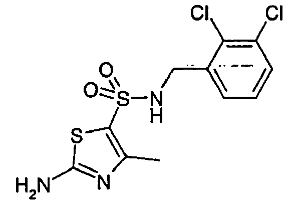

On chauffe 2,4 g de 2-acétamido-4-méthylthiazole 5-(N-2,3-dichlorobenzyl)sulfonamide, obtenu à l'étape 6.1, dans 100 ml d'une solution HCl 2N à 100 °C pendant 6h. On évapore le contenu du ballon puis on reprend le résidu au dichlorométhane. On lave trois fois avec une solution à 20% de carbonate de sodium et on sèche la phase organique sur du sulfate de magnésium anhydre. Après filtration et évaporation, on obtient 2g de poudre blanche.
RMN 300 MHz (DMSO) δ ppm : 2,24 (s,3H) ; 4,02 (d,2H) ; 7,31-7,60 (m,3H) ; 7,69 (s,2H) ; 8,28 (t,1H).

### Exemple 6.3: 2-{2-(S)-[2-(3,5-diftuorophényl)acétylamino]pentanoyl}-amino-4-méthylthiazole 5-(N-2,3-dichlorobenzyl)sulfonamide

A partir du 2-amino-4-méthylthiazole 5-(N-2,3-dichlorobenzyl)sulfonamide, obtenu à l'étape 6.2, 2 couplages successifs sont réalisés selon les procédés décrits aux étapes 1.4 et 1.5 de l'exemple 1.
On obtient 0,42g de produit final.
RMN : décrite dans le tableau ci-après. (composé n°64)
LC/MS : MH⁺ = 605

Le tableau qui suit illustre les structures chimiques et les propriétés physiques de quelques exemples de composés selon l'invention.

**Tableau 1**

| | | | | | | |
|---|---|---|---|---|---|---|
| 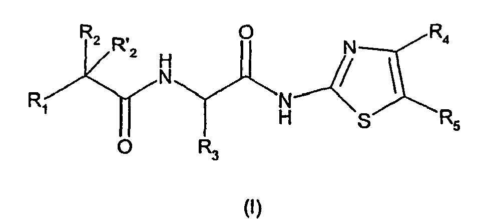 | | | | | | |

| **N°** | **R₁** | **R₂,R'₂** | **R₃** | **R₄** | **R₅** | **RMN** (DMSO d6 quand non précisé) * signifie 300MHz - ** signifie 360MHz - *** signifie 500MHz - ****signifie 600MHz |
|---|---|---|---|---|---|---|
| 1. | 3,5-difluorophényle | OH, H (S) | -(CH₂)₂CH₃ (S) | 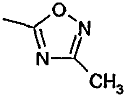 | -CH(CH₃)₂ | 89 (t,3H) ; 1,26-1,36 (m,2H) ; 1,39 (d,6H) ; 1,78 (m,2H) ; 2,48 (s,3H) ; 4,21 (m,1H); 4,53 (m,1H); 5,13 (d,1H); 6,63 (d,1H); 7,20 (m,3H) ; 8,34 (d,1H); 12,68 (s,1H)*** |
| 2. | 3,5-difluorophényle | OH, H | -(CH₂)₂CH₃ (S) | 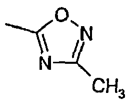 | -CH(CH₃)₂ | 0,84-0,90 (2t,3H) ; 1,25-1,35 (m,2H) ; 1,35 (d,6H); 1,76 (m,2H) ; 2,45 (s,3H) ; 4,17 (m,1H); 4,48 (m,1H); 5,11 et 5,13 (2a,1H); ( ,48 et 6,60 (2d,1H); 7,14-7,23 (m,3H) ; 8,32 et 8,35 (2d,1H); 12,69 (s,large,1H)*** |
| 3. | 3,5-difluorophényle | OH, H (S) | -(CH₂)₂CH₃ (S) | 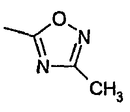 | 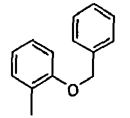 | 0,88 (t,3H) ; 1,24-1,34 (m,2H) ; 1,79 (m,2H) ; 2,31 (s,3H) ; 4,58 (m,1H) ; 5,09 (s,2H) ; 5,13 (m,1H) ; 6,63 (d,1H) ; 7,09-7,50 (m,12H); 8,37 (d,1H); 12,79 (s,large,1H)*** |
| 4. | 3,5-difluorophényle | OH, H (R) | -(CH₂)₂CH₃ (S) | 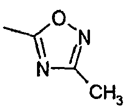 | 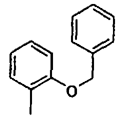 | 0,90 (t,3H) ; 1,27-1,40 (m,2H) ; 1,78 (m,2H) ; 2,30 (s,3H); 4,52 (m,1H, large) ; 5,07 (s,2H); 5,15 (d,1H); 6,48 (d,1H); 7,00-7,50 (m,12H) ; 8,40 (d,1H) ; 12,85 (s,1H,large)*** |
| 5. | 3,5-difluorophényle | OH, H (S) | -(CH₂)₂CH₃ (S) | 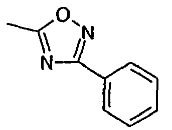 | -CH(CH₃)₂ | 0,91 (t,3H) ; 1,21-1,39 (m,2H) ; 1,47 (d,6H) ; 1,81 (m,2H) ; 4,32 (m,1H) ; 4,56 (m,1H); 5,16 (d,1H); 6,65 (d,1H); 7,22 (m,3H) ; 7,68 (m,3H) ; 8,16 (m,2H) ; 8,37 (d,1H); 12,76 (s, 1H,large)*** |
| 6. | 3,5-difluorophényle | OH, H(R) | -(CH₂)₂CH₃ (S) | 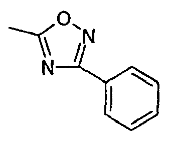 | -CH(CH₃)₂ | 0,92 (t,3H); 1,28-1,41 (m,2H) ; 1,43 (d,6H) ; 1,80 (m,2H) ; 4,31 (m,1H) ; 4,52 (m,1H) ; 5,17 (d,1H); 6,52 (d,1H); 7,19 (m,3H); 7,68 (m,3H) ; 8,15 (m,2H) ; 8,40 (d,1H) ; 12,80 (s,1H,large)*** |
| 7. | -C(CH₃)₃ | OH, H (S) | -(CH₂)₂CH₃ (S) | 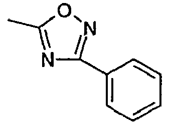 | -CH(CH₃)₂ | 0,93 (t,3H) ; 0,97 (s,9H); 1,31-1,42 (m,2H); 1,44 (m,6H) ; 1,77 (m,2H) ; 3,62 (d,1H); 4,30 (m,1H); 4,60 (m,1H); 5,62 (d,1H); 7,66 (m,3H); 7,88 (d,1H); 8,13 (m,2H); 12,71 (s,1H,large)*** |
| S. | -CH(CH₃)₂ | OH, H (S) | -(CH₂)₂CH₃ (S) | 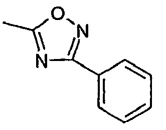 | -CH(CH₃)₂ | 0,85 (d,3H) ; 0,94 (t,3H); 0,98 (d,3H) ; 1,20-1,50 (m,8H); 1,78 (m,2H) ; 2,05 (m,1H); 3,80 (m,1H); 4,30 (m,1H); 4,61 (m,1H); 5,54 (d,1H); 7,66 (m,3H); 7,94 (d,1H) ; 8,14 (m,2H) ; 12,75 (s,1H,large)*** |
| 9. | 3,5-difluurophényle | H, H | -(CH₂)₂CH₃ (S) | -CN | -CH(CH₃)₂ | 0,95 (t,3H) ; 1,36-1,45 (m,8H); 1,69-1,75 (m,2H); 3,43 (m,1H); 3,64 (d,2H) ; 4,49 (m,1H); 7,00-7,20 (m,3H); 8,61 (d,1H); 12,69 (s,1H)*** |
| 10. | -C(CH₃)₃ | OH, H (S) | -(CH₂)₂CH₃ (S) | 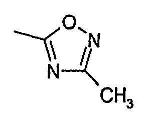 | 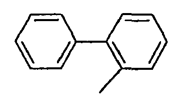 | 0,87 (t,3H) ; 0,90 (s,9H); 1,23-1,28 (m,2H); 1,70 (m,2H) ; 2,30 (s,3H) ; 3,56 (d,1H); 4,54 (s,large,1H) ; 5,61 (d,1H); 7,04 (d,2H) ; 7,18 (m,3H) ; 7,43-7,60 (m,4H) ; 7,83 (d,1H); |
| 11. | -C(CH₃)₃ | OH, H (S) | -(CH₂)₂CH3 (S) | 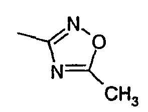 | -CH(CH₃)₂ | 12,63 (s,large,1H)*** 0,96 (t,3H) ; 1,01 (s,9H) ; 1,36-1,45 (m,8H); 1,79 (m,2H) ; 2,75 (s,3H) ; 3,65 (d,1H); 4,15 (m,1H) ; 4,62 (m,1H); 5,67 (d,1H) ; 7,90 (d,1H) ; 12,56 (s,1H,large)*** |
| 12. | -C(CH₃)₃ | OH, H (S) | -(CH₂)₂CH₃ (S) | 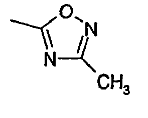 | 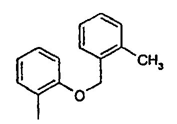 | 0,88 (t,3H) ; 0,91 (s,9H) ; 1,23-1,35 (m,2H) ; 1,73 (m,2H) ; 2,13 (s,3H); 2,23 (s,3H) ; 3,57 (d,1H); 4,57 (d,large,1H); 5,02 (s,2H); 5,58 (d,1H); 7,04-7,50 (m,8H) ; 8,41 (d,1H); 12,68 (s,large,1H)*** |
| 13. | -C(CH₃)₃ | OH, H (S) | -(CH₂)₂CH₃ (S) | 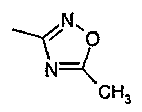 | 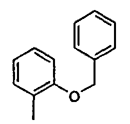 | 0,98 (t,3H) ; 1,03 (s,9H) ; 1,39-1,50 (m,2H); 1,84 (m,2H) ; 2,60 (s,3H) ; 3,68 (d,1H) ; 4,69 (m,1H); 5,16 (s,2H); 5,72 (d,1H); 7,00-7,60 (m,9H); 7,95 (d,1H); 12,71 (s,1H)*** |
| 14. | -C(CH₃)₃ | OH, H (S) | -(CH₂)₂CH₃ (S) | 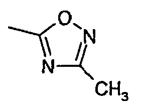 | 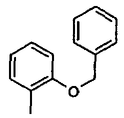 | 0,93 (t,3H) ; 0,98 (s,9H) ; 1,33-1,47 (m,2H) ; 1,80 (m,2H) ; 2,32 (s,3H) ; 3,63 (d,1H); 4,63 (m,1H); 5,12 (s,2H); 5,65 (d,1H); 7,00-7,52 (m,9H); 7,91 (d,1H); 12,87 (s,1H,large)*** |
| 15. | -C(CH₃)₃ | OH, H (S) | -(CH₂)₂CH₃ (S) | 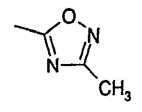 | 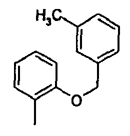 | 0,88 (t,3H) ; 0,91 (s,9H) ; 1,28-1,36 (m,2H); 1,73 (m,2H) ; 2,22 (s,3H) ; 2,26 (s,3H) ; 3,57 (d,1H); 4,57 (d,1H); 4,99 (s,2H) ; 5,59 (d,1H) 6,91 (m,2H) ; 7,06 (m,2H) ; 7,13 (m,1H); 7,16 (m,1H); 7,39-7,44 (m,2H); 7,90 (m,2H) 12,71 (s,large,1H)*** |
| 16. | -C(CH₃)₃ | OH, H (S) | -(CH₂)₂CH₃ (S) | 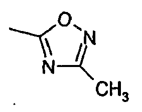 | 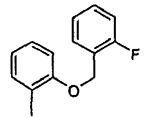 | 0,97 (t,3H) ; 0,99 (s,9H) ; 1,34-1,42 (m,2H) ; 1,70 (m,2H); 2,32 (s,3H) ; 3,65 (d,1H); 4,65 (d,1H) ; 5,15 (s,2H); 5,67 (d,1H) ; 7,10-7,55 (m,8H); 7,93 (d,1H); 12,74 (s,large,1H)*** |
| 17. | -C(CH₃)₃ | OH, H (S) | -(CH₂)₂CH₃ (S) | -CN | -CH(CH₃)₂ | 0,94 (t,3H) ; 0,98 (s,9H) ; 1,32-1,39 (m,2H) ; 1,42;(d,6H); 1,76 (m,2H) ; 3,43 (m,1H); 3,64 (d,1H); 4,60 (m,1H); 5,61 (d,1H) ; 7,60 (d,1H); 12,60 (s,1H,large)*** |
| 18. | 3,5-difluorophényle | OH, H | -(CH₂)₂CH₃ (S) | -CN | -CH(CH₃)₂ E et | 0,90 (m,3H) ; 1,28-1,39 (m,2H) ; 1,40 (m,6H) ; 3,44 (m,1H); 4,53 (m,1H); 5,16 (2d,1H); 49 et 6,61 (2d,1H) ; 7,19-7,34 (m,3H) ; 8,37 8,41 (2d,1H); 12,65 et 12,70 (2d,1H,large)*** |
| 19. | 3,5-difluorophényle | H, H | -(CH₂)₂CH₃ (S) | 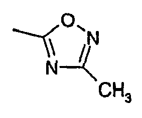 | 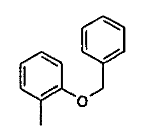 | 0,98 (t,3H) ; 1,39-1,83 (2m,4H) ; 2,35 (s,3H) ; 3,67 (m,1H); 4,57 (m,1H); 5,13 (s,2H); 7,02-7,54 (m,12H) ; 8,65 (d,1H); 12,9 (s,1H)*** |
| 20. | -C(CH₃)₃ | OH, H (S) | -(CH₂)₂CH₃ (S) | 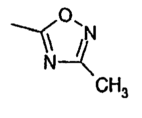 | 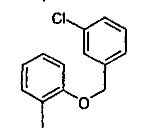 | 0,97 (t,3H) ; 1,01 (s,9H) ; 1,39-1,47 (m,2H) ; 1,81 (m,2H) ; 2,35 (s,3H); 3,66 (d,1H); 4,67 (d arge,1H); 5,15 (s,2H); 5,71 (d,1H); 7,15-7,55 (m,8H); 7,97 (d,1H); 12,86 (s,large,1H)*** |
| 21. | -C(CH₃)₃ | OH, H (S) | -(CH₂)₂CH₃ (S) | 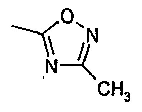 | 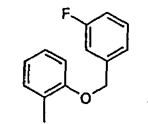 | 0,95 (t,3H) ; 0,99 (s,9H); 1,33-1,44 (m,2H) ; 1,80 (m,2H); 2,33 (s,3H); 3,65 (d,1H); 4,65 (s,large,1H); 5,10 (s,2H); 5,70 (d,1H); 6,96-7,53 (m,8H); 7,96 (d,1H); 12,88 (s,large,1H)*** |
| 22. | -C(CH₃)₃ | OH, H (S) | -(CH₂)₂CH₃ (S) | -CN | 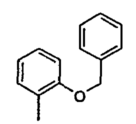 | 0,94 (t,3H) ; 1,04 (s,9H) ; 1,31-1,44 (m,2H) ; 1,77 (m,2H); 3,63 (d,1H) ; 4,62 (m,1H); 5,33 (s,2H); 5,62 (d,1H); 7,15-7,95 (m,10H); 12,70 (s,1H,large)*** |
| 23. | -C(CH₃)₃ | OH, H (S) | -(CH₂)₂CH₃ (S) | -CN | 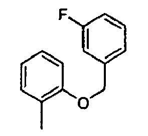 | 0,94 (t,3H) ; 0,98 (s,9H); 1,30-1,44 (m,2H) ; 1,77 (m,2H); 3,63 (d,1H); 4,62 (m,1H); 5,35 (s,2H); 5,62 (d,1H) ; 7,18-7,95 (m,9H); 12,72 (s,1H,large)*** |
| 24. | -C(CH₃)₃ | OH, H (S) | -(CH₂)₂CH₃ (S) | 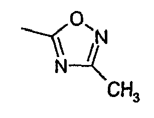 | 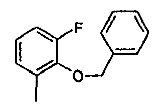 | 0,87 (t,3H) ; 0,89 (s,3H) ; 1,28-1,35 (m,2H) ; 1.75 (m,2H) ; 2,25 (s,3H) ; 3,57 (d,1H) ; 4,59 (m,1H); 4,86 (s,2H) ; 5,57 (d,1H); 6,98 (d,1H); 7,15-7,42 (m,7H); 7,86 (d,1H); 12,75 (s,large,1H)*** |
| 25. | 3,5-difluorophényle | OH, H (S) | -(CH₂)₂CH₃ (S) | 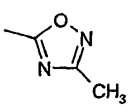 | 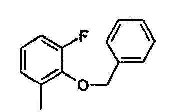 | 0,85 (t,3H) ; 1,21-1,33 (m,2H) ; 1,75 (m,2H) ; 2,48 (s,3H); 4,51 (m,1H) ; 4,86 (s,2H) ; 5,08 (d,1H) ; 6,55 (d,1H) ; 6,97-7,43 (m,13H); 8,32 (d,1H) ; 12,80 (s,1H)*** |
| 26. | 3,5-difluorophényle | OH, H (S) | -(CH₂)₂CH₃ (S) | 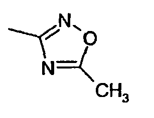 | 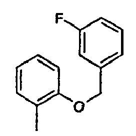 | 0,84 (t,3H) ; 1,21-1,31 (m,2H) ; 1,74 (m,2H); 2,48 (s,3H) ; 4,52 (m,1H); 5,00-5,10 (m,3H) ; 6,58 (d,1H) ; 6,96-7,40 (m,11H); 8,30 (d,1H) ; 12,68 (s,1H)*** |
| 27. | 3,5-difluorophényle | OH, H(R) | -(CH₂)₂CH₃ (S) | 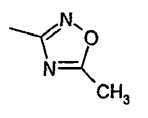 | 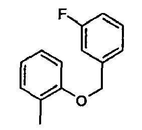 | 0,85 (t,3H) ; 1,25-1,35 (m,2H) ; 1,73 (m,2H) ; 2,48 (s,3H) ; 4,50 (m,1H) ; 5,07 (s,2H) 5,11 (d,1H) ; 6,43 (d,1H); 6,90-7,40 (m,11H); 8,35 (d,1H) ; 12,73 (s,1H)*** |
| 28. | -C(CH₃)₃ | OH, H (S) | -(CH₂)₂CH₃ (S) | 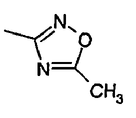 | 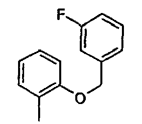 | 0,89 (t,3H) ; 1,32 (m,2H) ; 1,73 (m,2H) ; 2,48 (s,3H) ; 3,56 (d,1H); 5,07 (s,2H); 5,61 (d,1H) ; 6,90-7,40 (m,8H) ; 7,84 (d,1H) ; 12,63 (s,1H)*** |
| 29. | 3,5-difluorophényle | OH, H (S) | -(CH₂)₂CH₃ (S) | -CN | -CH(CH₃)₂ | 0,82 (t,3H) ; 1,20-1,27 (m,2H) ; 1,33 (d,6H) ; 1,70 (m,2H) ; 3,35 (m,1H); 4,47 (m,1H); 5,07 (d,1H); 6,54 (d,1H) ; 7,15 (m,3H) ; 8,29 (d,1H) ; 12,57 (s,1H)*** |
| 30. | 3,5-difluorophényle | OH, H (S) | -(CH₂)₂CH₃ (S) | -CN | 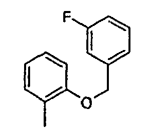 | 0,83 (t,3H); 1,20-1,30 (m,2H) ; 1,73 (m,2H) ; 4,51 (m,1H); 5,08 (d,1H); 5,29 (s,2H) ; 6,56 (d,1H); 7,12-7,68 (m,11H); 8,33 (d,1H); 12,71 (s,1H)*** |
| 31. | 3,5-difluorophényle | OH, H (S) | -(CH₂)₂CH₃ (S) | 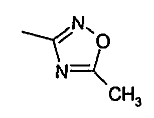 | 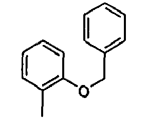 | 0,84 (t,3H) ; 1,25 (m,2H) ; 1,75 (m,2H) ; 4,51 (d,1H); 5,05 (s,2H) ; 5,08 (d,1H); 6,58 (d,1H) ; 6,90-7,40 (m,12H); 8,31 (d,1H) ; 12,65 (s,1H)*** |
| 32. | 3,5-difluorophényle | H, H | -(CH₂)₂CH₃ (S) | 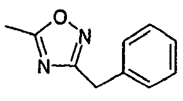 | H | 0,86 (t,3H) ; 1,28-1,37 (m,2H) ; 1,65 (m,2H) ; 3,57 (m,2H) ; 4,15 (s,2H) ; 4,45 (m,1H) ; 6,98 (d,2H) ; 7,07 (t,1H); 7,25 (t,1H); 7,34 (s,4H); 8,26 (s,1H) ; 8,54 (d,1H) ; 12,82 (s large,1H)**** |
| 33. | -C(CH₃)₃ | OH, H (S) | -(CH₂)₂CH₃ (S) | 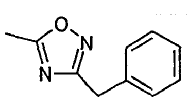 | H | 0,85 (t,3H); 0,91 (s,9H) ; 1,24 et 1,35 (2m,2H): 1,72 (m,2H); 3,56 (d,1H) ; 4,15 (s,2H); 4,56 (m,1H); 5,61 (d,1H); 7,26-7,35 (m,5H); 7,84 (d,1H); 8,28 (s,1H); 12,73 (s large,1H)**** |
| 34. | -C(CH₃)₃ | OH, H (S) | -(CH₂)₂CH₃ (S) | 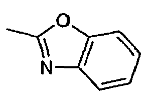 | -CH(CH₃)₂ | 0,88 (t,3H) ; 0,92 (s,9H) ; 1,28-1,35 (m,2H) ; 1,36 (d,6H) ; 1,72 (m,2H) ; 3,57 (d,1H); 4,35 (m,1H); 4,55 (m,1H); 5,60 (d,1H); 7,43 (m,2H); 7,77 (d,1H); 7,82 (m,2H); 12,60 |
| 35. | 3,5-difluorophényle | H, H | -(CH₂)₂CH₃ (S) | 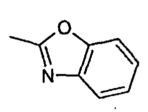 | -CH(CH₃)₂ | (s,H)*** 0,88 (t,3H) ; 1,31 (m,2H) ; 1,37 (d,6H) ; 1,67 (m,2H); 3,57 (m,2H) ; 4,35 (m,1H); 4,43 (m,1H); 6,98-7,10 (m,3H) ; 7,42 (m,2H) ; 7,75-7,83 (m,2H) ; 8,53 (d,1H); 12,64 |
| 36. | -C(CH₃)₃ | OH, H (S) | -(CH₂)₂CH₃ (S) | 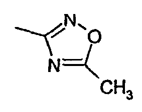 | 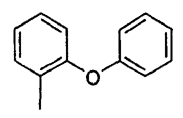 | (s,1H)*** 0,87 (t,3H) ; 0,91 (s,9H); 1,25-1,75 (m,4H) ; 2,50 (s,3H) ; 3,55 (d,1H) ; 4,55 (m,1H); 5,61 (d,1H) ; 6,81 (d,2H) ; 6,93 (d,1H); 7,06 (t,1H) ; 7,21 (t,1H); 7,30 (m,2H) ; 7,46 (m,2H); 7,82 (d,1H); 12,64 (s,1H)*** |
| 37. | 3,5-difluorophényle | H, H | -(CH₂)₂CH₃ (S) | 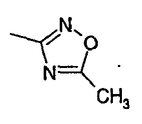 | 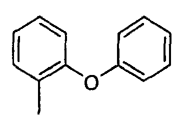 | 0,87 (t,3H); 1,25-1,75 (m,4H) ; 2,50 (s,3H) ; 3,56 (m,2H) ; 4,44 (m,1H) ; 6,80-7,50 (m,12H) ; 8,53 (d,1H) ; 12,73 (s,1H)*** |
| 38. | 3,5-difluorophényle | OH, H (S) | -(CH₂)₂CH₃ (S) | 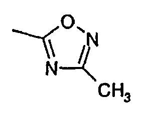 | 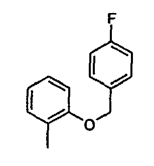 | 0,83 (t,3H); 1,22-1,30 (m,2H) ; 1,74 (m,2H); 2,25 (s,3H) ; 4,51 (m,1H) ; 5,01 (s,1H); 5,08 (d,1H); 6,57 (d,1H) ; 7,05-7,21 (m,9H) ; 7,40-7,45 (m,2H) ; 8,32 (d,1H); 12,75 (s large,1H)**** |
| 39. | 3,5-difluorophénylE | OH,H | -(CH₂)₂CH₃ (S) | H | 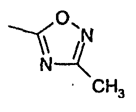 | 0,88 et 0,89 (2t,3H) ; 1,29 (m,2H) ; 1,78 (m,2H) ; 2,43 (s,3H) ; 4,58 (m,1H) ; 5,14 (m,1H) ; 6,47 et 6,61 (2d,1H) ; 7,13 (m,3H) ; 8,43 (s,1H) ; 8,39-8,47 (m,3H) ; 12,99 (s,large, 1H)*** |
| 40. | 3,5-difluorophényle | OH,H | -(CH₂)₂CH₃ (S) | CH₃ | 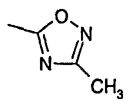 | 0,88 et 0,90 (2t,3H) ; 1,31 (m,2H) ; 1,79 (m,2H) ; 2,43 et 2,44 (2s,3H) ; 2,71 (s,3H) ; 4,57 (m,1H) ; 5,15 (m,1H) ; 6,48 et 6,63 (2d,1H) : 7,20 (m,3H) ; 8,40 et 8,46 (2d,1H) ; 12,90 (s,large, 1H)*** |
| 41. | -C(CH₃)₃ | OH, H (S) | -(CH₂)₂CH₃ (S) | H | 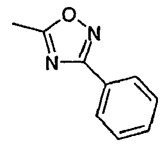 | 0,93 (t,3H) ; 0,96 (s,9H) ; 1,33-1,44 (m,2H) ; 1,78 (m,2H) ; 3,62 (d,1H) ; 4,66 (m,1H) ; 5,62 (d,1H) ; 7,65 (m,3H) ; 7,95 (d,1H) ; 8,10 (m,2H) ; 8,54 (s,1H) ; 13,00 (s,1H)*** |
| 42. | -CH(CH₃)₂ | OH, H (S) | -(CH₂)₂CH₃ (S) | H | 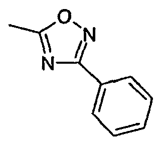 | 0,81 (m,3H) ; 0,92 (m,6H) ; 1,31-1,40 (m,2H) 1,75 (m,2H) ; 2,00 (m, 1H) ; 3,77 (m,1H) ; 4,63 (m,1H) ; 5,50 (d,1H) ; 7,61 (m,2H) ; 7,97 (d,1H) ; 8,07 (m,2H) ; 8,51 (s.1H) ; 12,99 (s,1H,large)*** |
| 43. | 3,5-difluorophényle | OH, H (S) | -(CH₂)₂CH₃ (S) | H | 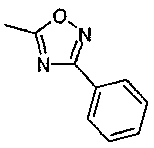 | 0,90 (t,3H) ; 1,25-1,40 (m,2H) ; 1,82 (m,2H) ; 4,63 (m, 1H) ; 5,16 (d,1H); 6,63 (d,1H); 7,18-7,22 (m,3H) ; 7,64-7,70 (m,3H) ; 8,12 (m,2H) 8,44 (m, 1H) ; 8,56 (s,1H) ; 13,05 (s,1HH)*** |
| 44. | -C(CH₃)₃ | OH, H (S) | -(CH₂)₂CH₃ (S) | CH₃ | 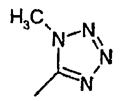 | 0,88 (t,3H) ; 0,91 (t,9H) ; 1,32 (m,2H) ; 1,71 (m,2H) ; 2,42 (s,3H) ; 3,56 (d,1H) ; 4,09 (s,3H) ; 4,57 (m,1H) ; 5,58 (d,1H) ; 7,84 (d,1H) ; 12,63 (s,large,1H)*** |
| 45. | 3,5-difluorophényle | H, H | -(CH₂)₂CH₃ (S) | CH₃ | 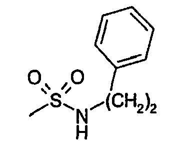 | 0,93 (t,3H) ; 1,40 (m,2H) ; 1,70 (m,2H) ; 2,46 (s,3H) ; 2,77 (m,2H) ; 3,14 (m,2H) ; 3,62 (d, 1H) ; 4,51 (m,1H) : 7,03-7,32 (m,8H); 7,99 (m,1H) ; 8,61 (d,1H) : 12,72 (s, large. 1H)*** |
| 46. | -C(CH₃)₃ | OH, H (S) | -(CH₂)₂CH₃ (S) | CH₃ | 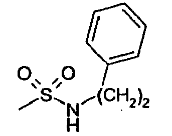 | 0,94 (t,3H) ; 0,98 (s,9H) ; 1,35 (m,2H) ; 1,76 (m,2H) ; 2,48 (s,3H) ; 2,78 (t,2H) ; 3,15 (m,2H) ; 3,63 (d,1H) ; 4,63 (m,1H) : 5,62 (d, 1H) ; 7,21-7,40 (m,5H); 7,91 (d,1H) : 8,01 (s,1H,large); 12,67 (s, 1H)*** |
| 47. | -C(CH₃)₃ | OH, H (S) | -(CH₂)₂CH₃ (S) | H | -CN | 0,95 (t,3H) ; 0,98 (t,9H) ; 1,39 (m,2H) ; 1,79 (m,2H) ; 3,64 (d, 1H) ; 4,64 (m, 1H) ; 5,65 (d,1H) ; 7,96 (m, 1H) 8,47 (s,1H) ; 13,15 (s, large, 1H)*** |
| 48. | -C(CH₃)₃ | OH, H (S) | -(CH_{z})₂CH₃ (S) | H | 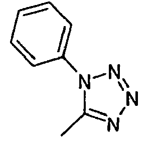 | 0,92 (t,3H) : 0,96 (t,9H) ; 1,33 (m,2H) ; 1,74 (m,2d) ; 3,61 (d, 1H) ; 4,61 (m, 1H) ; 5,64 (d,1H) ; 7,64 (s,1H) ; 7,74-7,82 (m, 5H) ; 7,92 (m,1H); 12,75 (s,large,1H)*** |
| 49. | 3,5-difluorophényle | OH, H (S) | -(CH₂)₂CH₃ (S) | H | 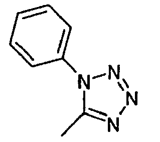 | 0,87 (t,3H) ; 1,28 (m,2H) ; 1,75 (m,2H) ; 4,55 (m, 1H) ; 5,12 (d,1H) ; 6,60 (d, 1H) ; 7,17-7,24 (m,3H) ; 7,64 (s,1H) ; 7,24-7,82 (m,5H) , 8,35 (d,1H) ; 12,79 (s,large,1H)*** |
| 50. | 3,5-difluorophényle | OH, H (R) | -(CH₂)₂CH₃ (S) | H | 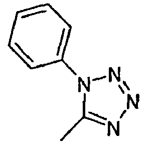 | 0,91 (t,3H) ; 1,32 (m,2H) ; 1,78 (m,2H) ; 4,58 (t,1H) ; 5,17 (m,1H) ; 6,50 (d,1H) ; 7,23 (m,3H) ;7,68 (s,1H) ; 7,77-7,86 (m,5H) ; 8/5 (m,1H); 12,85 (s,large,1H)*** |
| *51.* | -C(CH₃)₃ | OH, H (S) | -(CH₂)₂CH₃ (S) | H | 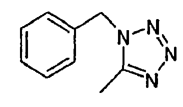 | 0,94 (t,3H) ; 0,97 (t,9H) ; 1,35 (m,2H) ; 1,77 (m,2M) ; 3,63 (m,1H) ; 4,65 (m,1H) ; 5,64 (d,1H) ; 6,00 (s,2H) ; 7,25 (m,2H) ; 7,40 (m,3H) ; 7,92 (d,1H) ; 8,17 (s,1H) ; 12,79 (s,1H,large)*** |
| 52. | 3,5-difluorophényle | H, H | -(CH₂)₂CH₃ (S) | CH₃ | 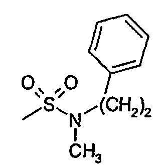 | 0,90 (t,3H) ; 1,33 (m,2H) ; 1,68 (m,2H) ; 2,47 (s,3H) : 2,80 (s,3H) ; 2,68 (t,2H) ; 3,30 (t,2H) : 3,59 (m,2H) : 4,46 (m,1H) ; 7,01-7,34 (m,8H) ; 8,55 (m,large, 1H) ; 12,84 (s,large,1H)*** |
| 53. | 3,5-difluorophényle | H, H | -(CH₂)₂CH₃ (S) | H | 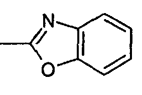 | 1,01 (t,3H) ; 1,42-1,60 (m,2H) ; 1,70-1,90 (m,2H) ; 3,71 (s,2H) ; 4,67 (m,1H) ; 7,12-7,30 (m,3H) ; 7,53 (m,2H) ; 7,88 (m,2H) ; 8,47 (s,1H) ; 8,75 (d,1H) ; 12,98 (s,1H, large)*** |
| 54. | 3,5-difluorophényle | OH, H | -(CH₂)₂CH₃ (S) | H | 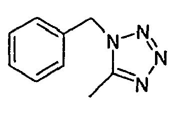 | 0,84 (t,3H) ; 1.22 (m,2H) ; 1,72 (m,2H) ; 4,51 (m,1H) ; 5,08 (m,1H) ; 5,94 (s,2H) ; 6,48 (m,1H) ; 7,12-7,19 (m,5H) ; 7,32-7,39 (m,3H) ; 8,1 (s,1H) ; 8,35 (m,1H) ; 12,77 (s,large,1H)*** |
| 55. | -C(CH₃)₃ | OH, H (S) | -(CH₂)₂CH₃ (S) | CH₃ | 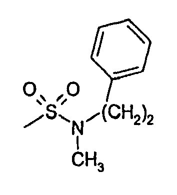 | 0,93 (t,3H) ; 0,98 (s,9H) ; 1,37 (m,2H) ; 1,76 (m,2H) ; 2,53 (s,3H) ; 2,84 (s,3H) ; 2,90 (t,2H) ; 3,33 (m,2H) ; 3,62 (d,1H) ; 4,59 (m,1H) ; 5,64 (d,1H) ; 7,26-7,45 (m,5H); 7,91 (m,1H) : 12,80 (s,large,1H)*** |
| 56. | -C(CH₃)₃ | OH, H (S) | -(CH₂)₂CH₃ (S) | CH₃ | 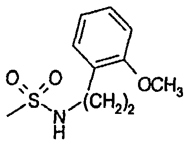 | 0,87 (t,3H) ; 0,90 (s,9H) ; 1,20 (m,2H) ; 1,69 (m,2H) ; 2,40 (s,3H) ; 2,66 (t,2H) ; 3,04 (m,2H) ; 3,56 (d,1H) ; 3,70 (s,3H) ; 4,54 (m,1H) 5,54 (d, 1H) ; 6,80-7,20 (m,4H) ; 7 4 (d,1H) ; 7,92 (s,large, 1H) ; 12,60 (s,large,1H)*** |
| 57. | 3,5-difluorophényle | H, H | -(CH₂)₂CH₃ (S) | CH₃ | 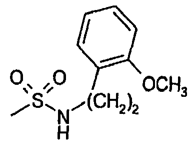 | 0,93 (t,3H) ; 1,30-1,50 (m,2H) ; 1,73 (m,2H) ; 2,45 (s,3H) ; 2,72 (t,2H) ; 3,11 (m,2H) ; 3,63 (s,2H) ; 3,74 (s,3H) ; 4,51 (m,1H) ; 6,80-7,40 (m,7H) ; 7,97 (m,1H) ; 8,62 (d, 1H) ; 12,72 (s,1H)*** |
| 58. | 3,5-difluorophényle | H, H | -(CH₂)₂CH₃ (S) | CH₃ | 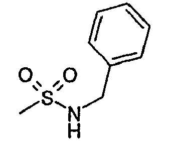 | 0,94 (t,3H) ; 1,32-1,44 (m,2H) ; 1,71 (m,2H) ; 2,46 (s,3H) ; 3,63 (m,2H) ; 4,51 (m, 1H) ; 7,05-7,33 (m,8H) ; 8,48 (t, 1H) ; 8,63 (d, 1H) ; 12,70 (s,1H,large)*** |
| 59. | -C(CH₃)₃ | OH, H (S) | -(CH₂)₂CH₃ (S) | CH₃ | 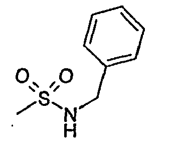 | 0,96 (t,3H) ; 1,00 (s,9H) ; 1,33-1,47 (m,2H) ; 1,78 (m,2H) ; 2,48 (s,3H) ; 3,65 (d,1H) ; 4,17 (d,2H) ; 4,63 (q,1H) ; 5,64 (d,1H) ; 7,30 (m,5H) ; 7,93 (m,1H) ; 8,51 (s, 1H,large) ; 12,50 (s,1H, large)*** |
| 60. | 3,5-difluorophényle | H, H | CH₃(CH₂)₂- (S) | H | 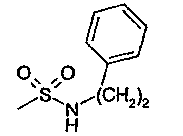 | 0,85 (t,3H) ; 1,34 (m,2H) ; 1,63 (m,2H) ; 2,70 (t,2H) ; 3,04 (m,2H) ; 3,55 (s,2H) ; 4,46 (m, 1H) ; 6,96-7,30 (m,8H) ; 7,85 (s, 1H) ; 7,92 (t,1H) ; 8,57 (d,1H) ; 12,80 (s,1H)*** |
| 61. | -C(CH₃)₃ | OH,H (S) | -(CH₂)₂CH₃ (S) | CH₃ | 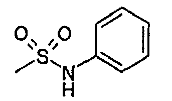 | 0,84 (t,3H) ; 0,88 (s,9H) ; 1,23 (m,2H) ; 1,65 (m,2H) ; 2,27 (s,3H) ; 3,53 (d, 1H) ; 4,49 (m, 1H) ; 5,53 (d, 1H) ; 7,00-7,30 (m,5H) ; 7,81 (d, 1H) ; 10,40 (s,1H) ; 12,59 (s,1H)*** |
| 62. | 3,5-difluorophényle | OH, H (S) | -(CH₂)₂CH₃ (S) | CH₃ | 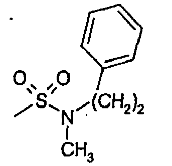 | 0,82 (t,3H) ; 1,18-1,28 (m,2H) ; 1,71 (m,2H) ; 2,46 (s,3H) ; 2,77 (s,3H) ; 2,83 (m,2H) ; 2,72 (m,2H) ; 4,48 (m,1H) ; 5,07 (d,1H) ; 6,54 (d,1H) ; 7,10-7,70 (m,8H) ; 8,31 (m, 1H) ; 12,75 (s,1H)*** |
| 63. | 3,5-difluorophényle | H, H | CH₃(CH₂)₂- (S) | CH₃ | 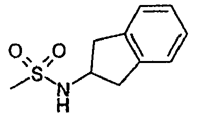 | 0,87 (t, 3H) ; 1,15-1,30 (m,2H) ; 1,66 (m,2H) ; 2,48 (s,3H) ; 2,79 (m,2H) ; 3,03 (m,2H) ; 3,56 (m,2H) ; 3,97 (m,1H) ; 4,46 (m,1H) ; 6,97-7,17 (m,7H) ; 8,29 (d,1H) ; 8,57 (d,1H) ; 12,72 (s, 1H)*** |
| 64. | 3,5-difluorophényle | H, H | CH₃(CH₂)₂- (S) | CH₃ | 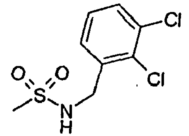 | 0,87 (t,3H) ; 1,25-1,35 (m,2H) ; 1,62-1,68 (m,2H) ; 2,41 (s,3H) ; 3,56 (m,2H) ; 4,22 (m,2H) ; 4,43 (m,1H) ; 6,99 (m,2H) ; 7,08 (m,1H) ; 7,28 (m, 1H) ; 7,39 (m, 1H) ; 7,50 (m,1H) ; 8,56 (m,2H) ; 12,68 (s,1H)*** |
| 65. | -C(CH₃)₃ | OH, H (S) | CH₃(CH₂)₂- (S) | H | 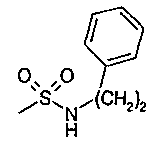 | 0,87 (t,3H) ; 0,90 (s,9H) ; 1,25-1,28 (m,2H) ; 1,70 (m,2H) ; 2,72 (t,2H) ; 3,06 (m,2H) ; 3,57 (d, 1H) ; 4,57 (m,1H) ; 5,54 (d, 1H) ; 7,18 (m,3H) ; 7,25 (m,2H) ; 7,86 (m, 1H) ; 7,89 (s, 1H) ; 7,94 (m,1H) ; 12,74 (s, 1H)*** |
| 66. | -C(CH₃)₃ | OH, H (S) | CH₃(CH₂)₂- (S) | CH₃ | 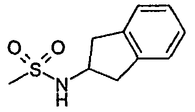 | 0,87 (t,3H) ; 0,91 (s,9H) ; 1,20-1,35 (m,2H) ; 1,69 (m,2H) ; 2,48 (s,3H) ; 2,79 (m,2H) ; 3,03 (m,2H) ; 3,56 (d,1H) ; 3,98 (m,1H) ; 4,56 (m,1H) ; 5,57 (d,1H) ; 7,10-7,17 (m,4H) ; 7,86 (m,1H); 8,31 (m, 1H); 12,63 (s,1H)*** |
| 67. | 3,5-difluorophényle | OH, H (S) | CH₃(CH₂)₂- (S) | H | 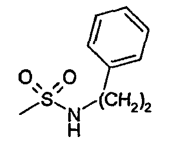 | 0,81 (t,3H) ; 1,17-1,30 (m,2H) ; 1,70 (m,2H) ; 2,70 (m,2H) ; 3,04 (s,3H) ; 4,50 (m,1H) ; 5,06 (m, 1H) ; 6,52 (d,1H) ; 7,11-7,24 (m, 8H) ; 7,87 (s, 1H) ; 7,95 (m,1H) ; 8,33 (d, 1H) ; 12,76 (s,1H)*** |
| 68. | 3,5-difluorophényle | OH, H (R) | CH₃(CH₂)₂-(S) | H | 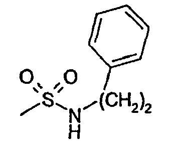 | 0,84 (t,3H) ; 1,22-1,34 (m,2H) ; 1,72 (m,2H) ; 2,71 (m,2H) ; 3,05 (m,2H) ; 4,49 (m,1H) ; 5,10 (d, 1H) ; 6,43 (d,1H) ; 7,12-7,25 (m,8H) ; 7,87 (s,1H) ; 7,94 (m,1H) ; 8,39 (d, 1H) ; 12,80 (s,1H,large)*** |
| 69. | 3,5-difluorophényle | OH, H (S) | CH₃(CH₂)₂- (S) | - CH₃ | 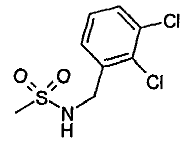 | 0,83 (t,3H) ; 1,18-1,29 (m,2H) ; 1,71 (m,2H) ; 2,41 (s,3H) ; 4,23 (m,2H) ; 4,49 (m,1H) ; 5,08 (d, 1H) ; 6,55 (d, 1H) ; 7,15 (m,3H) ; 7,29 (t,1H) ; 7,39 (d, 1H) ; 7,51 (d, 1H) ; 8,33 (d, 1H) ; 8,61 (t, 1H) ; 12,64 (s, 1H)*** |
| 70. | 3,5-difluorophényle | OH, H (R) | CH₃(CH₂)₂- (S) | CH₃ | 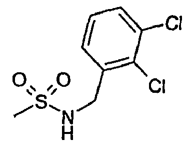 | 0,82 (T,3H) ; 1,20-1,30 (m,2H) ; 1,69 (m,2H) ; 2,39 (s,3H) ; 4,20 (m,2H) ; 4,44 (m, 1H) ; 5,08 (d,1H) ; 6,41 (d,1H) 7,12 (m,3H) 7,2 (t, 1H) 7,36 (d, 1H) ; 7,47 (d,1H) ; 8,36 (d, 1H) ; 8,58 (m, 1H) ; 12,66 (s, 1H)*** |
| 71. | 3,5-difluorophényle | H, H | CH₃(CH₂)₂- (S) | CH₃ | 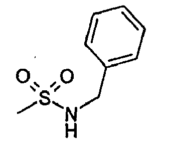 | 0,94 (t,3H) ; 1,30-1,44 (m,2H) ; 1,69-1,76 (m,2H) ; 2,46 (s,3H) ; 3,63 (m,2H) ; 4,14 (m,2H) ; 4,51 (m,1H) ; 7,06 (m,2H) ; 7,15 (m, 1H) ; 7,25-7,33 (m,5H) ; 8,48 (t, 1H) ; 8,63 (d, 1H) ; 12,70 (s,1H,large)*** |
| 72. | 3,5-difluorophényle | H, H | CH₃(CH₂),- (S) | CH₃ | 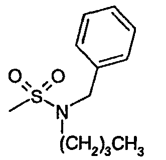 | 0,69 (t,3H) ; 0,87 (t,3H) ; 1,09 (m,2H) ; 1,27 (m,2H) ; 1,35 (m,2H) ; 1,66 (m,2H) ; 3,10 (m,2H) ; 3,56 (m,2H) ; 4,34 (s,2H) ; 4,44 (m,1H) ; 6,98 (m,2H) ; 7,08 (m, 1H) ; 7,25-7,36 (m, 5H) ; 8,59 (d, 1H) ; 12,82 (s, 1H)**** |
| 73. | -C(CH₃)₃ | OH, H (S) | CH₃(CH₂)₂- (S) | CH₃ | 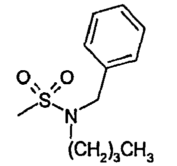 | 0,69 (t,3H) ; 0,87 (t,3H) ; 0,91 (s,9H) ; 1,09 (m,2H) ; 1,26 (m,2H) ; 1,70 (m,2H) ; 3,11 (m,2H) ; 3,56 (d,1H) ; 4,35 (s,2H) ; 4,55 (m,1H) ; 5,57 (d, 1H) ; 7,27-7,36 (m,5H); 7,86 (s,1H) ; 12, 73 (s,1H)**** |
| 74. | 3,5-difluorophényle | H, H | CH₃(CH₂)₂- (S) | CH₃ | 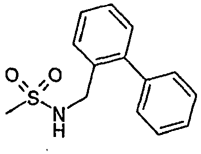 | 0,88 (t,3H) ; 1,25-1,35 (m,2H) ; 1,66 (m,2H) 2,28 (s,3H) ; 3,57 (m,2H) ; 3,99 (d,2H) ; 4,45 (m,1H) ; 6,99-7,45 (m,12H) ; 8,32 (t, 1H) ; 8,58 (d,1H) ; 12,64 (s,1H)**** |
| 75. | -C(CH₃)₃ | OH, H (S) | CH₃(CH₂)₂- (S) | CH₃ | 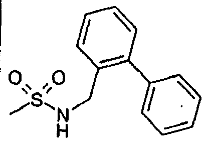 | 0,88 (t,3H) ; 0,91 (s,9H) ; 1,25-1,35 (m,2H) ; 1,70 (m,2H) ; 2,29 (s,3H) ; 3,57 (d, 1H) ; 4,00 (d,2H) ; 5,55 (d,1H) ; 7,15-7,45 (m,9H) ; 7.86 (d, 1H) ; 8,33 (t,1H) ; 12,56 (s, 1H)**** |
| 76. | -CH(CH₃)₂ | OH, H (S) | CH₃(CH₂)₂- (S) | CH₃ | 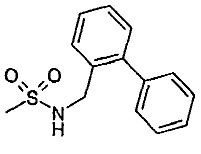 | 0,79 (d,3H) ; 0,89 (d,3H) ; 0,90 (t,3H) ; 1,20-1,40 (m,2H) ; 1,71 (m,2H) ; 1,98 (m,1H) ; 2,29 (s,3H) ; 3,75 (m,1H) ; 4,00 (d,2H) ; 4,56 (m, 1H) ; 5,47 (d,1H) ; 7,15-7,50 (m,9H) 7,91 (d,1H) ; 8,33 (t,1H) 12,59 (s,1H)**** |
| 77. | -C(CH₃)₃ | OH, H (S) | CH₃(CH₂)₂- (S) | CH₃ | 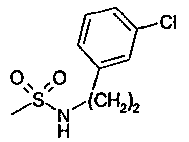 | 0,87 (t,3H) ; 0,90 (s,9H) ; 1,20-1,35 (m,2H) ; 1,68 (m,2H) ; 2,59 (s,3H) ; 2,72 (m,2H) ; 3,11 (m,2H) ; 3,56 (d, 1H) ; 4,55 (m, 1H) ; 5,55 (d, 1H) ; 7,11-7,27 (m,4H) ; 7,84 (d, 1H) 7,94 (m, 1H) ; 12,60 (s, 1H)**** |
| 78. | 3,5-difluorophényle | H, H | CH₃(CH₂)₂- (S) | CH₃ | 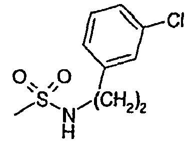 | 0,86 (t,3H) ; 1,25-1,35 (m,2H); 1,65 (m,2H) ; 2,38 (s,3H) ; 2,71 (m,2H) ; 3,11 (m,2H) ; 3,56 (m,2H) ; 4,45 (m,1H) ; 6,90-7,30 (m,7H) ; 7,93 (m, 1H) ; 8,57 (d,1H) ; 12,68 (s,1H)**** |
| 79. | -CH(CH₃)₂ | OH, H (S) | CH₃(CH₂)₂- (S) | CH₃ | 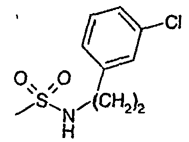 ; | 0,77 (d,3H) ; 0,86 (t,3H) ; 0,91 (d,3H) ; 1,24-1,35 (m,2H) ; 1,70 (m,2H) ; 1,95 (m, 1H) ; 2,38 (s,3H) ; 2,72 (m,2H) ; 3,11 (m,2H) ; 3,73 (m,1H) ; 4,55 (m,1H) ; 5,47 (d,1H) ; 7,11-7,27 (m,4H) ; 7,89 (d, 1H) ; 7,94 (q,1H) 12,63 (s,1H)**** |
| 80. | -C(CH₃)₃ | OH, H (S) | CH₃(CH₂)₂- (S) | CF₃ | 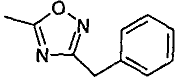 | 0,87 (t,3H) ; 0,90 (s,9H) ;1,27-1,37 (m,2H) ; 1,73 (m, 2H) ; 3,57 (d,2H) ;4,18 (s,2H) ; 4,58 (m,1H) ; 5,52 (d,1H) ; 7,26 -7,34 (m,SH) ; 7,90 (d, 1H) ; 13,29 (s, 1H) **** |
| | | | | | | |

| N° | R₁ | R₂,R'₂ | R₃ | R₄ | Rₛ | MH+ |
|---|---|---|---|---|---|---|
| 81. | -C(CH₃)₃ | OH, H (S) | -(CH₂)₂CH₃ (S) | 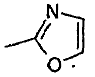 | 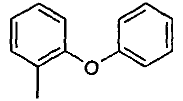 | 549 |
| 82. | 3,5-difluorophényle | H, H | -(CH₂)₂CH₃ (S) | 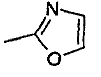 | 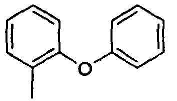 | 589 |
| 83. | -C(CH₃)₃ | OH, H (S) | -(CH₂)₂CH₃ (S) | 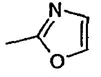 | -CH(CH₃)₂ | 423 |
| 84. | 3,5-difluorophényle | H, H | -(CH₂)₂CH₃ (S) | 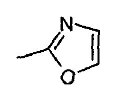 | -CH(CH₃)₂ | 463 |
| 85. | 3,5-difluorophényle | H, H | -CH₂CH₃ (S) | CH₃ | 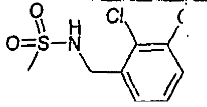 | 591 |
| 86. | -C(CH₃)₃ | OH, H (S) | -CH₂CH₃ (S) | CH₃ | 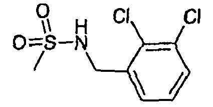 | 551 |
| 87. | -CH(CH₃)₂ | OH, H (S) | -CH₂cH₃ (S) | CH₃ | 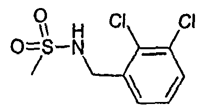 | 537 |
| 88. | 3,5-difluorophényle | H, H | -CH₃ (S) | CH₃ | 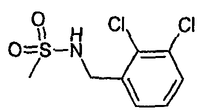 | 577 |
| 89. | -C(CH₃)₃ | OH, H (S) | -CH₃ (S) | CH₃ | 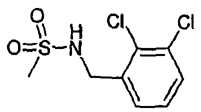 | 537 |
| 90. | -CH(CH₃)₂ | OH, H (S) | -CH₃ (S) | CH₃ | 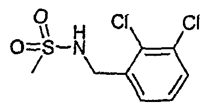 | 523 |
| 91. | -C(CH₃)₃ | OH, H (S) | -(CH₂)₂CH, (S) | CH₃ | 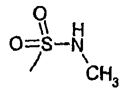 | 421 |
| 92. | 3,5-difluorophényle | H, H | -(CH₂)₂cH₃ (S) | CH₃ | 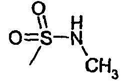 | 461 |
| 93. | 3,5-difluorophényle | OH, H | -(CH₂)₂CH₃ (S) | CH₃ | 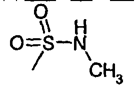 | 477 |
| 94. | 3,5-difluorophényle | H, H | -(CH₂)₂CH₃ (S) | CH₃ | 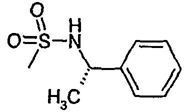 | 551 |
| 95. | 3,5-difluorophényle | H, H | -(CH₂)₂CH₃ (S) | CH₃ | 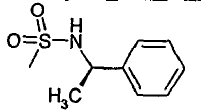 | 551 |
| 96. | -C(CH₃)₃ | OH, H (S) | -(CH₂)₂CH₃ (S) | CH₃ | 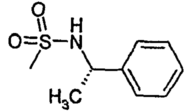 | 511 |
| 97. | -C(CH₃)₃ | OH, H (S) | -(CH₂)₂CH₃ (S) | CH₃ - | 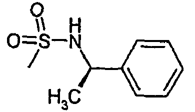 | 511 |
| 98. | 3,5-difluorophényle | OH, H (S) | -(CH₂)₂CH₃ (S) | CH₃ | 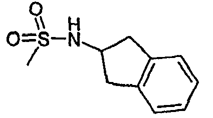 | 579 |
| 99. | 3,5-difluorophényle | OH, H (R) | -(CH₂)₂CH₃ (S) | CH₃ | 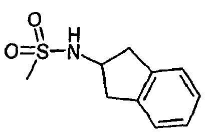 | 579 |
| 100. | -CH(CH₃)₂ | OH, H (S) | -(CH₂)₂CH₃ (S) | CH₃ | 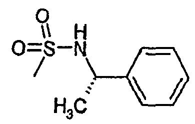 | 497 |
| 101. | -C(CH₃)₃ | OH, H (S) | -(CH₂)₂CH₃ (S) | CH₃ | 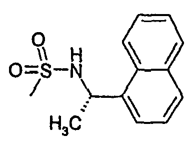 | 561 |
| 102. | 3,5-difluorophényle | H, H | -(CH₂)₂CH) (S) | CH₃ | 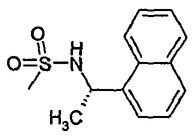 | 601 |
| 103. | 3,5-difluorophényle | OH, H | -(CH₂)₂CH₃ (S) | CH₃ | 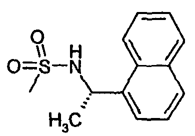 | 617 |
| 104. | -C(CH₃)₃ | OH, H (S) | -(CH₂)₂CH₃ (S) | CH₃ | 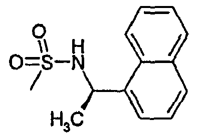 | 561 |
| 105. | 3,5-difluorophényle | H, H | -(CH₂)₂CH₃ (S) | CH₃ | 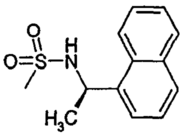 | 601 |
| 106. | 3,5-difluorophényle | OH, H | -(CH₂)₂CH₃ (S) | CH₃ | 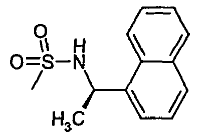 | 617 |
| 107. | -C(CH₃)₃ | OH, H (S) | -(CH₂)₂CH₃ (S) | CH₃ | 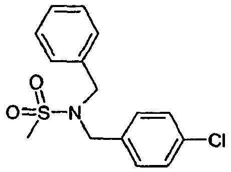 | 621 |
| 148. | 3,5-difluorophényle | H, H | -(CH₂)₂CH₃ (S) | CH₃ | 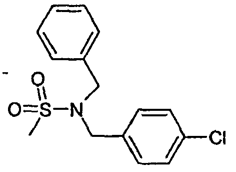 | 661 |
| 109. | -C(CH₃)₃ | OH. H (S) | -(CH₂)₂CH₃ (S) | CH₃ | 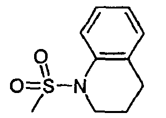 | 637 |
| 110. | 3,5-difluorophényle | H. H | -(CH₂)CH₃ (S) | CH₃ | 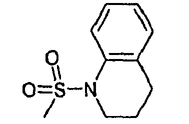 | 563 |
| 111. | 3,5-difluorophényle | OH, H (S) | -(CH₂)₂CH₃ (S) | CH₃ | 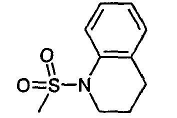 | 579 |
| 112. | 3,5-difluorophényle | OH, H (R) | -(CH₂)₂CH₃ (S) | CH₃ | 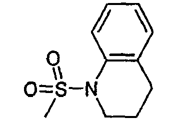 | 579 |
| 113. | -C(CH₃)₃ | OH, H (S) | -(CH₂)₂CH₃ (S) | CH₃ | 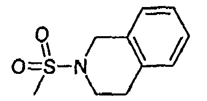 | 523 |
| 114. | 3,5-difluorophényle | H, H | -(CH₂)₂CH₃ (S) | CH₃ | 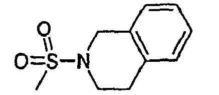 | 563 |
| 115. | 3,5-difluorophényle | OH, H (S) | -(CH₂)₂CH₃ (S) | CH₃ | 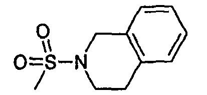 | 579 |
| 116. | 3,5-difluorophényle | OH, H (R) | -(CH₂)₂CH₃ (S) | CH₃ | 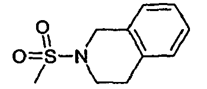 | 579 |
| 117. | 3,5-difluorophényle | OH, H (S) | -(CH₂)₂CH₃ (S) | CH₃ | 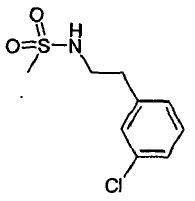 | 601 |
| 118. | 3,5-difluorophényle | OH, H (R) | **-**(CH₂)₂CH₃ (S) | CH₃ | 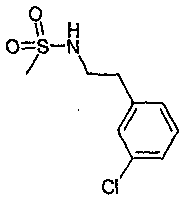 | 601 |
| 119. | -C(CH₃)₃ | OH, H (S) | -(CH₂)₂CH₃ (S) | CH₃ | 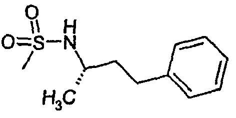 | 539 |
| 120. | -C(CH₃)₃ | OH, H (S) | -(CH₂)₂CH₃ (S) | CH₃ | 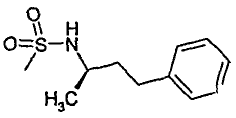 | 539 |
| 121. | 3,5-difluorophényle | H, H | -(CH₂)₂CH₃ (S) | CH₃ | 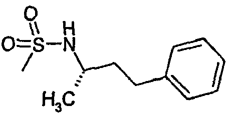 | 579 |
| 122. | 3,5-difluorophényle | H, H | -(CH₂)₂CH₃ (S) | CH₃ | 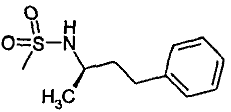 | 579 |
| 123. | 3,5-difluorophényle | OH, H (S) | -(CH₂)₂CH₃ (S) | CH₃ | 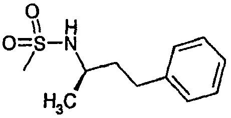 | 595 |
| 124. | 3,5-difluorophényle | OH, H | -(CH₂)₂cH₃ (S) | CH₃ | 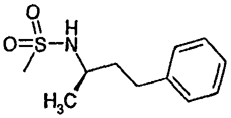 | 595 |
| | | | | | | |

| N° | R₁ | R₂,R'₂ | R₃ | R₄ | R₅ | RMN (DMSO d6 quand non précisé) * signifie 300MHz - ** signifie 360MHz - *** signifie 500MHz - ****signifié 600MHz |
|---|---|---|---|---|---|---|
| 125. | -C(CH₃)₃ | OH, H (S) | -(CH₂)₂CH₃ (S) | 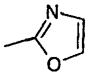 | 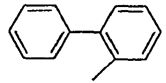 | 0,86 (t,3H) ; 0,90 (s,9H) ; 1,30 (m,2H) ; 1,68 (m,2H) ; 3,55 (d,1H) ; 4,54 (m,1H) ; 5,59 (d,1H) ; 6,80 (s,1H) ; 7,04-7,57 (m,9H) ; 7,77 (d,1H) ; 8,19 (s,1H) ; 12,41 |
| 126. | -CH(CH₃)₂ | OH, H (S) | -(CH₂)₂CH₃ (S) | 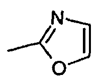 | 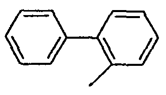 | (s,large,1H )**** 0,77 et 0,90 (2d,6H) ; 0,87 (t,9H) ; 1,24 (m,2H) ; 1,69 (m,2H) ; 1,97 (m,2H) ; 3,78 (m,1H) ; 4,54 (m,1H) ; 5,48 (d,1H) ; 6,80 (s, 1H) ; 7,04-7,56 (m,9H) ; 7,82 (d, 1H) ; |
| 127. | 3,5-difluorophényle | H, H | -(CH₂)₂CH₃ (S) | 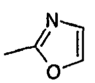 | 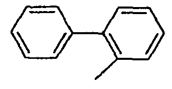 | 8,18 (s,1H) ; 12,44 (s,large,1H)**** 0,87 (t,3H) ; 1,30 (m,2H) ; 1,65 (m,2H) ; 3,56 (m,2H) ; 4,44 (m,1H) ; 6,79 (s,1H) ; 6,97-7,56 (m,12H) ; 8,18 (s,1H) ; 8,48 (d,1H): 12,51 (s, 1H)**** |
| 128. | -C(CH₃)₃ | OH, H (S) | -(CH₂)₂CH₃ (S) | 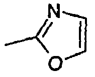 | 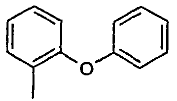 | 0,87 (t,3H) ; 0,91 (s,9H) ; 1,28 (m,2H) ; 1,70 (m,2H) ; 3,55 (d, 1H) ; 4,56 (m,1H) ; 5,58 (d,1H) ; 6,79-7,50 (m, 10H) ; 7,78 (d, 1H) ; 8,32 (s, 1H) ; 12,52 (s,1H)**** |
| 129. | -C(CH₃)₃ | OH, H (S) | -(CH₂)₂CH₃ (S) | 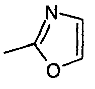 | 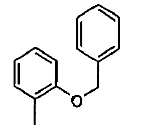 | 0,88 (t,3H) ; 0,92 (s,9H) ;1,30 (m,2H) 1,72 (m,2H) ; 3,57 (d, 1H) ; 4,58 (m,1H) ; 5,08 (s,2H) ; 5,59 (d,1H) ; 7,02-7,43 (m, 10H) ; 7,81 (d, 1H) ; 8,25 (s, 1H) ; 12,50 (s, 1H)**** |
| 130. | 3,5-difluorophényle | H, H | -(CH₂)₂CH₃ (S) | 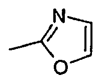 | 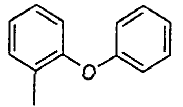 | 0,87 (t,3H) ; 1,32 (m,2H) ; 1,65 (m,2H) ; 3,56 (m,2H) ; 4,46 (m,1H) ; 6,79-7,50 (m, 13H) ; 8,32 (s, 1H) ; 8,49 (d, 1H) ; 12,58 (s, 1H)**** |
| 131. | -C(CH₃)₃ | OH, H (S) | -(CH₂)₂CH₃ (S) | 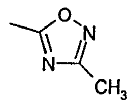 | 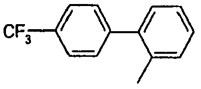 | 0,88 (t,3H) ; 0,91 (s,9H) ; 1,30 (m,2H) ; 1,70 (m,2H) ; 2,24 (s,3H) ; 3,57 (d,1H) ; 4,56 (m, 1H) ; 5,58 (d, 1H) ; 7,23-7,63 (m,8H) ; 7,83 (d,1H) ; 8,25 (s, 1H) ; 12,65 (s, 1H)**** |
| 132. | -CH(CH₃)₂ | OH, H (S) | -(CH₂)₂CH₃ (S) | 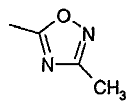 | 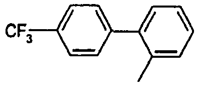 | 0,78 et 0,91 (2d,6H) ; 0,88 (t,3H) ; 1,28 (m,2H) ; 1,72 (m,2H) ; 1,98 (m, 1H) ; 2,23 (s,3H) ; 3,75 (m, 1H) ; 4,56 (m, 1H) ; 5,47 (d,1H) ; 7,23-7,63 (m,8H) ; 7,88 (d,1H) ; |
| 133. | -C(CH₃)₃ | OH, H (S) | -(CH₂)₂CH₃ (S) | 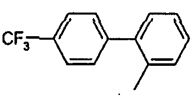 | 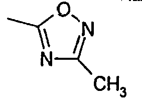 | 12,69 (s,1H)**** 0,87 (t,3H) ; 0,90 (s,9H) ; 1,31 (m,2H) ; 9,70 (m,2H) ; 2,20 (s,3H) ; 3,56 (d,1H) ; 4,57 (m, 1H) ; 5,55 (d, 1H) ; 7,17 (d,2H) ; 7,50-7,65 (m,6H) ; 7,86 (d, 1H) ; 12,87 (s, 1H, |
| 134. | -CH(CH₃)₂ | OH, H (S) | -(CH₂)₂CH₃ (S) | 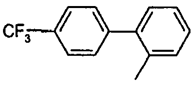 | 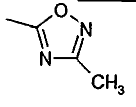 | large)**** 0,78 et 0,91 (2d,6H) ; 0,87 (t,3H) ; 1,30 (m,2H) ; 1,71 (m,2H) ; 1,98 (m, 1H) ; 2,20 (s,3H) ; 3,74 (m, 1H) ; 4,57 (m,1H) ; 5,45 (d,1H) ; 7,18 (d,2H) ; 7,50-7,65 (m,6H) ; 7,90 (d,1H) ; 12,90 (s,1H, large)**** |
| 135. | 3,5-difluorophényle | H, H | -(CH₂)₂CH₃ (S) | 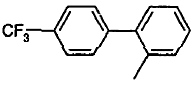 | 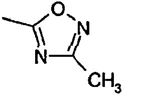 | 0,86 (t,3H) ; 1,34 (m,2H) ; 1,66 (m,2H) ; 2,20 (s,3H) ; 3,55 (m,2H) ; 4,48 (m,1H) : 6,98 (d,2H) ; 7,07 (t, 1H) ; 7,17 (d,2H) ; 7,47-7,65 (m,6H) ; 8,59 (d,1H) ; 12,96 (s,1H)**** |
| 136. | -CH(CH₃)₂ | OH, H (S) | -(CH₂)₂CH₃ (S) | 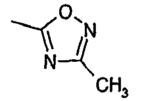 | 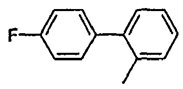 | 0,83-0,99 (m,9H) ; 1,23-1,43 (m,2H) ; 1,78 (m,2H) ; 2,05 (m,1H) ; 2,31 (s,3H) ; 3,81 (m,1H) ; 4,61 (m,1H) ; 5,57 (d,1H) ; 7,11 (m,4H) ; 7,48-7,6( (m,4H) ; 7,96 (d, 1H) ; 12,80 (s,1H)**** |
| 137. | -CH(CH₃)₂ | OH, H (S) | -(CH₂)₂CH₃ (S) | 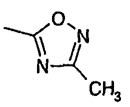 | 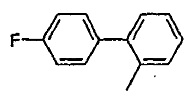 | 0,88 (t,3H) ; 0,92 (s,9H) ; 1,24-1,41 (m,2H) ; 1,72 (m,2H) ;1,99 (s,3H) ; 3,57 (d,1H) ; 4,55 (m,1H) ; 5,63 (d,1H) ; 7,10-7,20 (m,4H) ; 7,40-7,60 (m,4H), 7,86 (d,1H) ; 12,67 (s 1H)**** |

Dans le tableau :
- (S) ou (R) dans les colonnes « R₃ » et «R₂, R_{2'}» indiquent la stéréochimie du carbone asymétrique, portant R₃ ou R₂, dans la formule (I). Pour le carbone portant R₂, l'indication (S) ou (R) ne concerne pas le cas où R₂ et R_{2'} forment ensemble un groupe oxo ;
- MH+ est la valeur de la masse du composé protonné par un atome d'hydrogène (masse du composé + 1), déterminée par LC-MS.

Les composés de l'invention ont fait l'objet d'essais pharmacologiques qui ont montré leur intérêt comme substances actives en thérapeutique.

Ils ont en particulier été testés quant à leurs effets d'inhibition de la production du peptide β-amyloïde (β-A4).

Le peptide β-amyloïde (β-A4) est un fragment d'une protéine précurseur plus importante appelée APP (Amyloid Precursor Protein). Cette dernière est produite et présente dans différentes cellules de tissu animal ou humain. Toutefois, son clivage, dans le tissu cérébral, par des enzymes de type protéase, conduit à la formation du peptide β-A4 qui s'accumule sous forme de plaque amyloïde. Les deux protéases responsables de la production du peptide amyloide sont connues sous le nom de beta et gamma-secrétases (Wolfe MS, Secretase targets for Alzheimer's disease: identification and therapeutic potential, J Med Chem. 2001, 44(13):2039-60).

Or il a été démontré que ce dépôt progressif du peptide β-A4 est neurotoxique et pourrait jouer un rôle important dans la maladie d'Alzheimer.

Ainsi, les composés de la présente invention, en tant qu'inhibiteur de la production du peptide β-amyloïde (β-A4) par inhibition de la gamma sécrétase, peuvent être utilisés dans le traitement de pathologies comme la démence sénile, la maladie d'Alzheimer, le syndrome de Down, la maladie de Parkinson, l'angiopathie amyloïde et/ou les désordres cérébro-vasculaires, les démences fronto-temporales et la maladie de Pick, les démences post-traumatiques, les pathologies liées à des processus de neuro-inflammation, la maladie de Huntington et le syndrome de Korsakov.

Les tests ont été réalisés selon le protocole décrit ci-après.

Pour l'essai cellulaire β-amyloïde, la lignée CHO-K1 coexprimant le CT100 de APP et PS1 M146L clone 30-12 est utilisée. La lignée cible l'inhibition de gamma secrétase. La préséniline est liée à l'activité gamma-secrétase (Wolfe MS, Haass C., The Role of presenilins in gamma-secretase activity, J Biol Chem. 2001, 276(8):5413-6) et sa coexpression avec la protéine amyloïde ou son fragment N-terminal entraîne une augmentation de sécrétion du peptide A1-42 (β-A4) générant ainsi un outil pharmacologique permettant d'évaluer l'inhibition par les composés de formule (1) de la production du peptide β-A4. L'ensemencement des plaques de culture de 96 puits est réalisé à raison de 1x10⁵ cellules par puits dans 150µl de milieu d'incubation. La présence d'un pourcentage minimal (1,3% final) de sérum permet l'adhésion cellulaire au plastique après 2-3 heures d'incubation à 37°C, en présence de 5% CO₂., Les produits (15µl) sont testés à 10µM DMSO 1% final et sont incubés durant 24-25h à 37°C en présence de 5% CO₂ et de 100% d'humidité. Après cette incubation de 24-25h, les surnageants cellulaires (100µl) sont transférés dans les plaques ELISA, traitées avec l'anticorps de capture 6E10 (6E10, épitope : aa1-17, INTERCHIM/SENETEK 320-10), pour déterminer le taux de peptides amyloïdes sécrété par les cellules en présence de composés selon l'invention. Une gamme de peptide contrôle, « peptide 1-40 », synthétique à 5 et 10 ng/ml est traitée parallèlement. Les plaques ELISA sont incubées pendant une nuit à 4°C.

La quantité de peptide fixé est détectée de façon indirecte en présence d'un compétiteur correspondant au peptide tronqué, le peptide 1-28 couplé à la biotine qui est ensuite détectée par la streptavidine couplée à la phosphatase alcaline. Le substrat, le p-Nitrophényl Phosphate (pNPP *FAST* p-Nitrophényl Phosphate, Sigma N2770), donne un produit de réaction soluble jaune lisible à 405nm. La réaction est stoppée avec une solution d'EDTA 0,1 M. Pour cela, après fixation du peptide amyloïde dans la plaque ELISA, 50µl de peptide 1-28 biotinylé sont ajoutés aux 100µl de surnageant cellulaire et incubés 30 minutes à température ambiante. Les plaques ELISA sont ensuite lavées 3 fois. Après séchage par inversion sur papier absorbant, 100µl de streptavidine-Alcaline Phosphatase (Interchim/Jackson ImmunoResearch Laboratories 016-050-084), sont ajoutés par puits et incubés 1 heure à température ambiante. Les plaques sont à nouveau lavées puis le substrat de la phosphatase alcaline (pNPP 1mg/ml) est ajouté à raison de 100µl par puits. Après une incubation de 30 minutes à température ambiante, la réaction est stoppée par ajout de 100µl par puits d'EDTA 0,1 M et la lecture est effectuée à 405nm.

Les composés de formule (I) selon la présente invention les plus actifs ont montré une CE₅₀ (concentration effectrice à 50%) inférieure à 500 nM, plus particulièrement inférieure à 100 nM.
Le tableau 2 ci-dessous présente les CE₅₀ de quelques composés selon l'invention.

**Tableau 2**

| Composé N° | CE₅₀ (nM) |
|---|---|
| 7 | 52 |
| 34 | 42 |
| 40 | 85 |
| 51 | 94 |

Les résultats des tests biologiques montrent que les composés sont des inhibiteurs de la formation du peptide p-amyloïde (β-A4).
Ainsi, ces composés peuvent être employés dans le traitement des pathologies dans lesquelles un inhibiteur de la formation du peptide β-amyloïde (β-A4) apporte un bénéfice thérapeutique. Notamment de telles pathologies sont la démence sénile, la maladie d'Alzheimer, le syndrome de Down, la maladie de Parkinson, l'angiopathie amyloïde, les désordres cérébro-vasculaires, les démences fronto-temporales et la maladie de Pick, les démences post-traumatiques, les pathologies liées à des processus de neuro-inflammation, la maladie de Huntington et le syndrome de Korsakov. L'utilisation des composés selon l'invention pour la préparation d'un médicament destiné à traiter les pathologies ci-dessus mentionnées fait partie intégrante de l'invention.

L'invention a également pour objet des médicaments qui comprennent un composé de formule (I), ou un sel d'addition de ce dernier à un acide pharmaceutiquement acceptable ou encore un hydrate ou un solvat du composé de formule (I). Ces médicaments trouvent leur emploi en thérapeutique, notamment dans le traitement des pathologies ci-dessus mentionnées.

Selon un autre de ses aspects, la présente invention concerne des compositions pharmaceutiques renfermant en tant que principe actif, au moins un composé selon l'invention. Ces compositions pharmaceutiques contiennent une dose efficace d'un composé selon l'invention, ou d'un sel pharmaceutiquement acceptable, d'un hydrate ou d'un solvat dudit composé, et éventuellement un ou plusieurs excipients pharmaceutiquement acceptables.
Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intraveineuse, topique, locale, intratrachéale, intranasale, transdermique ou rectale, le principe actif de formule (I) ci-dessus, son sel, son solvat ou son hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules, les chewing-gums et les solutions ou suspensions orales, les formes d'administration sublinguale, buccale, intratrachéale, intraoculaire, intranasale, par inhalation, les formes d'administration sous-cutanée, intramusculaire ou intraveineuse et les formes d'administration rectale ou vaginale. Pour l'application topique, on peut utiliser les composés selon l'invention dans des crèmes, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Afin d'obtenir l'effet prophylactique ou thérapeutique désiré, la dose de principe actif peut varier entre 0,1 mg et 200 mg par kg de poids du corps et par jour. Bien que ces dosages soient des exemples de situation moyenne, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.

Chaque dose unitaire peut contenir de 0,1 à 1000 mg, de préférence de 0,1 à 500 mg, de principe actif en combinaison avec un ou plusieurs excipients pharmaceutiques. Cette dose unitaire peut être administrée 1 à 5 fois par jour de façon à administrer un dosage journalier de 0,5 à 5000 mg, de préférence de 0,5 à 2500 mg.

## Revendications

1. Composé répondant à la formule générale (I) : 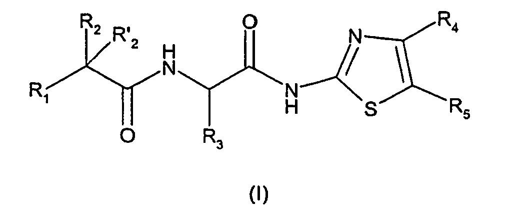 dans laquelle,
R₁ représente soit un C₁₋₆ alkyle éventuellement substitué par un à trois substituants choisis parmi un halogène, un trifluorométhyle, un hydroxy, un C₁₋₆ alcoxy, un C₁₋₆ thioalkyle, un thiophène ou un phényle ; soit un C₃₋₇ cycloalkyle, un thiophène, un benzothiophène, un pyridinyle, un furanyle ou un phényle ; les dits groupes phényle étant éventuellement substitués par un à trois substituants choisis parmi un atome d'halogène, un C₁₋₆ alkyle, un C₁₋₆ alcoxy, un hydroxy, un méthylènedioxy, un phénoxy, un benzyloxy ou un trifluorométhyle ;
R₂ et R'₂ représentent indépendamment l'un de l'autre un atome d'hydrogène, un atome d'halogène, un hydroxy, un C₁₋₃ alcoxy, un C₁₋₃ alkyle, un C₃₋₇ cycloalkyle, un groupe O-C(O)-C₁₋₆ alkyle, ou R₂ et R'₂ forment ensemble un groupe oxo ;
R₃ représente soit un atome d'hydrogène, soit un C₁₋₆ alkyle éventuellement substitué par un hydroxy, un C₁₋₆ cycloalkyle ou un C₁₋₃ alcoxy ;
R₄ et R₅ représentent, indépendamment l'un de l'autre un atome d'hydrogène, un C₁₋₇ alkyle, un trifluorométhyle, un groupe L ou un groupe Z ; 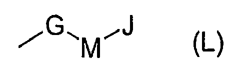 G représente un C₁₋₇ alkyle ou une liaison simple ;
M représente un C₃₋₇ cycloalkyle, un phényle, un naphtyle ou un pyridinyle, le groupe M étant éventuellement substitué par un ou plusieurs groupes choisis parmi un atome d'halogène, un groupe hydroxy, un C₁₋₃ alkyle, un C₁₋₃ alcoxy,
un trifluorométhyle, un trifluorométhoxy, un -O-CHF₂ ;
J représente un atome d'hydrogène ou un groupe -Y-K ;
Y représente une liaison simple, un atome d'oxygène, de soufre, un groupe -C₁₋₄ alkylène-, -O-C₁₋₄ alkylène-, -C₁₋₄ alkylène-O- ou -N(W)-, le groupe -C₁₋₄ alkylène- étant éventuellement substitué par un groupe hydroxy ou C₁₋₃ alcoxy ;
W représente soit un atome d'hydrogène, soit un C₁₋₃ alkyle éventuellement substitué par un phényle, soit un phényle ;
K représente un groupe phényle ou pyridinyle, le groupe K étant éventuellement substitué par un ou plusieurs groupes choisis parmi un atome d'halogène, un groupe hydroxy, un C₁₋₃ alkyle, un C₁₋₃ alcoxy, un trifluorométhyle, un trifluorométhoxy, un -O-CHF₂;
à la condition qu'au moins un groupe R₄ ou R₅ représente un groupe Z ;
Z représente un groupe CN, SO₂NR₆R₇, ou un groupe hétéroaromatique ; ledit groupe hétéroaromatique étant éventuellement substitué par un groupe R₈ ; R₈ représentant soit un C₁₋₄ alkyle lui-même éventuellement substitué par un CN, un phényle ou un phénoxy ; soit un phényle ; les dits groupes phényle et phénoxy étant éventuellement substitués par 1 à 3 groupes choisis parmi un atome d'halogène, un C₁₋₃ alkyle, un C₁₋₃ alcoxy, un trifluorométhyle ;
R₆ et R₇ représentent, indépendamment l'un de l'autre, soit un atome d'hydrogène, soit un groupe C₁₋₆ alkyle éventuellement substitué par un C₃₋₇ cycloalkyle, un C₃₋₇ cycloalkènyle, C₁₋₃ alcoxy, un phényle, un naphtalènyle, un morpholinyle ou un pyridinyle ; soit un C₃₋₇ cycloalkyle, C₁₋₆ alcoxy, un phényle ou un indanyle ; les dits groupés C₃₋₇ cycloalkyle, C₃₋₇ cycloalkènyle, phényle, naphtalènyle, morpholinyle, pyridinyle et indanyle étant éventuellement substitués par un ou deux groupes choisis parmi un C₁₋₃ alkyle, un hydroxy, un C₁₋₃ alcoxy, un phényle ou un atome d'halogène ; ou
R₆ et R₇, avec l'atome d'azote qui les porte, forment un cycle aziridine, azétidine, pyrrolidine, pipéridine, morpholine ou benzopipéridine ;
à l'état de base, de sel d'addition à un acide, d'hydrate ou de solvat.

2. Composé selon la revendication 1, **caractérisé en ce que** :
R₁ représente un C₁₋₆ alkyle ou un phényle éventuellement substitué par 1 à 3 atomes d'halogène ;
R₂ et R'₂ représentent, indépendamment l'un de l'autre, un atome d'hydrogène ou un hydroxy ;
R₃ représente un C₁₋₆ alkyle ;
R₄ et R₅ représentent, indépendamment l'un de l'autre un atome d'hydrogène,
un C₁₋₇ alkyle, un trifluorométhyle, un groupe L ou un groupe Z ; 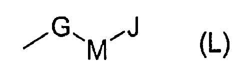
G représente un C₁₋₇ alkyle ou une liaison simple ;
M représente un phényle éventuellement substitué par un ou plusieurs atomes d'halogène ;
J représente un atome d'hydrogène ou un groupe -Y-K ;
Y représente une liaison simple, un atome d'oxygène, -O-C₁₋₄ alkylène- ;
K représente un groupe phényle éventuellement substitué par un ou plusieurs groupes choisis parmi un atome d'halogène, un C₁₋₃ alkyle, un trifluorométhyle ;
à la condition qu'au moins un groupe R₄ ou R₅ représente un groupe Z ;
Z représente un groupe CN, SO₂NR₆R₇, ou un groupe hétéroaromatique ; ledit groupe hétéroaromatique étant éventuellement substitué par un groupe R₈ ; R₈ représentant soit un C₁₋₄ alkyle lui-même éventuellement substitué par un phényle ; soit un phényle ;
R₆ et R₇ représentent, indépendamment l'un de l'autre, soit un atome d'hydrogène, soit un groupe C₁₋₆ alkyle éventuellement substitué par un phényle ou par un naphtalènyle ; soit un phényle ou un indanyle ; les dits groupes phényle étant éventuellement substitués par un ou deux groupes choisis parmi un C₁₋₃ alcoxy, un phényle ou un atome d'halogène ; ou
R₆ et R₇, avec l'atome d'azote qui les porte, forment un cycle benzopipéridine ;
à l'état de base, de sel d'addition à un acide, d'hydrate ou de solvat.

3. Composé de formule (I) selon la revendication 1 ou 2, **caractérisé en ce que** :
R₁ représente un C₁₋₄ alkyle, de préférence un isopropyle ou un ter-butyle, ou un phényle substitué par deux atomes de fluor ;
R₂ et R'₂ représentent, indépendamment l'un de l'autre, un atome d'hydrogène ou un hydroxy ;
R₃ représente un C₁₋₄ alkyle, de préférence un méthyle, éthyle ou propyle ;
à l'état de base, de sel d'addition à un acide, d'hydrate ou de solvat.

4. Procédé de préparation d'un composé de formule (I) selon l'une quelconque des revendications 1 à 3, comprenant l'étape consistant à
réaliser un couplage peptidique du 2-amino-thiazole de formule (III) 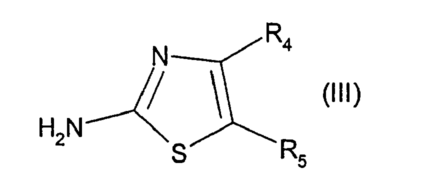 avec l'acylaminoacide de formule (II) 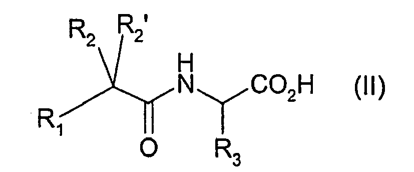 dans lesquelles R₁, R₂, R'₂, R₃, R₄, R₅ sont tels que définis dans la formule (I) selon l'une quelconque des revendications 1 à 3.

5. Procédé de préparation d'un composé de formule (I) selon l'une quelconque des revendications 1 à 3, comprenant l'étape consistant à réaliser un couplage peptidique du composé de formule (IV) 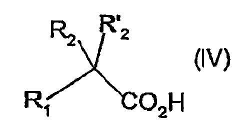 avec l'amine de formule (VI) 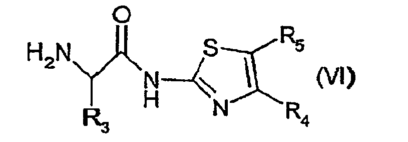 dans lesquelles R₁, R₂, R'₂, R₃, R₄, R₅ sont tels que définis dans la formule (I) selon l'une quelconque des revendications 1 à 3.

6. Médicament **caractérisé en ce qu'**il comprend un composé de formule (I) selon l'une quelconque des revendications 1 à 3, à l'état de base, de sel, d'hydrate ou de solvat pharmaceutiquement acceptable.

7. Composition pharmaceutique contenant au moins un composé de formule (I) selon l'une quelconque des revendications 1 à 3, à l'état de base, de sel, d'hydrate ou de solvat pharmaceutiquement acceptable et éventuellement un ou plusieurs excipients pharmaceutiquement acceptables.

8. Composé de formule (I) selon l'une quelconque des revendications 1 à 3, à l'état de base, de sel, d'hydrate ou dé solvat pharmaceutiquement acceptable, pour son utilisation pour traiter une pathologie dans laquelle un inhibiteur de la formation du peptide β-amyloïde β-A4 apporte un bénéfice thérapeutique.

9. Composé de formule (I) selon l'une quelconque des revendications 1 à 3, à l'état de base, de sel, d'hydrate ou de solvat pharmaceutiquement acceptable, pour son utilisation pour traiter la démence sénile, la maladie d'Alzheimer, le syndrome de Down, la maladie de Parkinson, l'angiopathie amyloïde, les désordres cérébro-vasculaires, les démences fronto-temporales et la maladie de Pick, les démences post-traumatiques, les pathologies liées à des processus de neuro-inflammation, la maladie de Huntington et/ou le syndrome de Korsakov.

## Claims

1. Compound conforming to the general formula (I) : 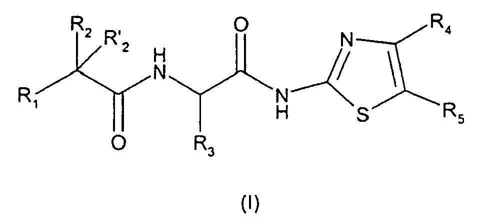 in which
R₁ represents either a C₁₋₆ alkyl optionally substituted by one to three substituents selected from a halogen, a trifluoromethyl, a hydroxyl, a C₁₋₆ alkoxy, a C₁₋₆ thioalkyl, a thiophene or a phenyl; or a C₃₋₇ cycloalkyl, a thiophene, a benzothiophene, a pyridinyl, a furanyl or a phenyl; the said phenyl groups being optionally substituted by one to three substituents selected from a halogen atom, a C₁₋₆ alkyl, a C₁₋₆ alkoxy, a hydroxyl, a methylenedioxy, a phenoxy, a benzyloxy or a trifluoromethyl;
R₂ and R'₂ represent independently of one another a hydrogen atom, a halogen atom, a hydroxyl, a C₁₋₃ alkoxy, a C₁₋₃ alkyl, a C₃₋₇ cycloalkyl or an O-C(O)-C₁₋₆ alkyl group, or R₂ and R'₂ together form an oxo group;
R₃ represents either a hydrogen atom or a C₁₋₆ alkyl optionally substituted by a hydroxyl, a C₁₋₆ cycloalkyl or a C₁₋₃ alkoxy;
R₄ and R₅ represent independently of one another a hydrogen atom, a C₁₋₇ alkyl, a trifluoromethyl, a group L or a group Z; 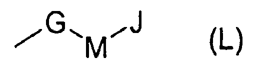 G represents a C₁₋₇ alkyl or a single bond;
M represents a C₃₋₇ cycloalkyl, a phenyl, a naphthyl or a pyridinyl, the group M being optionally substituted by one or more groups selected from a halogen atom, a hydroxyl group, a C₁₋₃ alkyl, a C₁₋₃ alkoxy, a trifluoromethyl, a trifluoromethoxy and a -O-CHF₂;
J represents a hydrogen atom or a group -Y-K;
Y represents a single bond, an oxygen or sulphur atom, a -C₁₋₄ alkylene- , -O-C₁₋₄ alkylene- or -C₁₋₄ alkylene-O-group or a group -N(W)-, the -C₁₋₄ alkylene- group being optionally substituted by a hydroxyl or C₁₋₃ alkoxy group;
W represents either a hydrogen atom, or a C₁₋₃ alkyl optionally substituted by a phenyl, or a phenyl;
K represents a phenyl or pyridinyl group, the group K being optionally substituted by one or more groups selected from a halogen atom, a hydroxyl group, a C₁₋₃ alkyl, a C₁₋₃ alkoxy, a trifluoromethyl, a trifluoromethoxy and an -O-CHF₂;
with the proviso that at least one group R₄ or R₅ represents a group Z;
Z represents a CN group, a group SO₂NR₆R₇ or a heteroaromatic group, the said heteroaromatic group being optionally substituted by a group R₈; R₈ representing either a C₁₋₄ alkyl which is itself optionally substituted by a CN, a phenyl or a phenoxy; or a phenyl; the said phenyl and phenoxy groups being optionally substituted by 1 to 3 groups selected from a halogen atom, a C₁₋₃ alkyl, a C₁₋₃ alkoxy and a trifluoromethyl;
R₆ and R₇ represent, independently of one another, either a hydrogen atom, or a C₁₋₆ alkyl group optionally substituted by a C₃₋₇ cycloalkyl, a C₃₋₇ cycloalkenyl, C₁₋₃ alkoxy, a phenyl, a naphthalenyl, a morpholinyl or a pyridinyl; or a C₃₋₇ cycloalkyl, C₁₋₆ alkoxy, a phenyl or an indanyl; the said C₃₋₇ cycloalkyl, C₃₋₇ cycloalkenyl, phenyl, naphthalenyl, morpholinyl, pyridinyl and indanyl groups being optionally substituted by one or two groups selected from a C₁₋₃ alkyl, a hydroxyl, a C₁₋₃ alkoxy, a phenyl or a halogen atom; or
R₆ and R₇ with the nitrogen atom which carries them form an aziridine, azetidine, pyrrolidine, piperidine, morpholine or benzopiperidine ring;
in the form of a base, addition salt with an acid, hydrate or solvate.

2. Compound according to Claim 1, **characterized in that**:
R₁ represents a C₁₋₆ alkyl or a phenyl which is optionally substituted by 1 to 3 halogen atoms;
R₂ and R'₂ represent independently of one another a hydrogen atom or a hydroxyl;
R₃ represents a C₁₋₆ alkyl;
R₄ and R₅ represent independently of one another a hydrogen atom, a C₁₋₇ alkyl, a trifluoromethyl, a group L or a group Z; 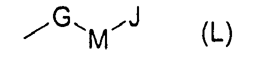
G represents a C₁₋₇ alkyl or a single bond;
M represents a phenyl which is optionally substituted by one or more halogen atoms;
J represents a hydrogen atom or a group -Y-K;
Y represents a single bond, an oxygen atom or -O-C₁₋₄ alkylene-;
K represents a phenyl group which is optionally substituted by one or more groups selected from a halogen atom, a C₁₋₃ alkyl and a trifluoromethyl;
with the proviso that at least one group R₄ or R₅ represents a group Z;
Z represents a CN group, a group SO₂NR₆R₇ or a heteroaromatic group, the said heteroaromatic group being optionally substituted by a group R₈; R₈ representing either a C₁₋₄ alkyl which is itself optionally substituted by a phenyl; or a phenyl;
R₆ and R₇ represent, independently of one another, either a hydrogen atom, or a C₁₋₆ alkyl group optionally substituted by a phenyl or by a naphthalenyl; or a phenyl or an indanyl; the said phenyl groups being optionally substituted by one or two groups selected from a C₁₋₃ alkoxy, a phenyl or a halogen atom; or
R₆ and R₇, with the nitrogen atom which carries them, form a benzopiperidine ring;
in the form of a base, addition salt with an acid, hydrate or solvate.

3. Compound of formula (I) according to Claim 1 or 2, **characterized in that**:
R₁ represents a C₁₋₄ alkyl, preferably an isopropyl or a tert-butyl, or a phenyl substituted by two fluorine atoms;
R₂ and R'₂ represent independently of one another a hydrogen atom or a hydroxyl;
R₃ represents a C₁₋₄ alkyl, preferably a methyl, ethyl or propyl;
in the form of a base, addition salt with an acid, hydrate or solvate.

4. Process for preparing a compound of formula (I) according to any one of Claims 1 to 3, comprising the step consisting in
carrying out a peptide coupling of the 2-aminothiazole of formula (III) 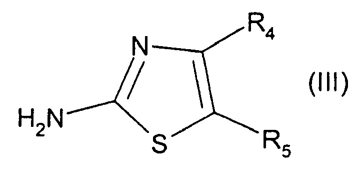 with the acylamino acid of formula (II) 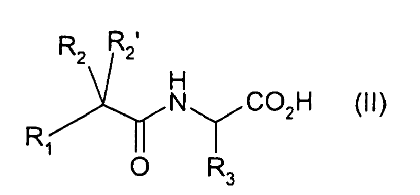 in which R₁, R₂, R'₂ , R₃ , R₄ and R₅ are as defined in the formula (I) according to any one of Claims 1 to 3.

5. Process for preparing a compound of formula (I) according to any one of Claims 1 to 3, comprising the step consisting in
carrying out a peptide coupling of the compound of formula (IV) 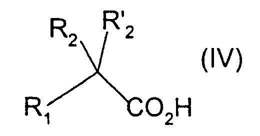 with the amine of formula (VI) 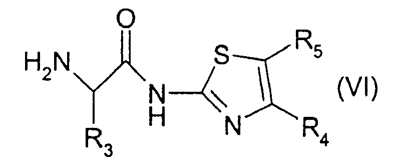 in which R₁, R₂, R'₂, R₃, R₄ and R₅ are as defined in the formula (I) according to any one of Claims 1 to 3.

6. Medicinal product **characterized in that** it comprises a compound of formula (I) according to any one of Claims 1 to 3, in the form of a pharmaceutically acceptable base, salt, hydrate or solvate.

7. Pharmaceutical composition comprising at least one compound of formula (I) according to any one of Claims 1 to 3, in the form of a pharmaceutically acceptable base, salt, hydrate or solvate, and optionally one or more pharmaceutically acceptable excipients.

8. Compound of formula (I) according to any one of Claims 1 to 3, in the form of a pharmaceutically acceptable base, salt, hydrate or solvate, for use in the treatment of a pathology in which a β-A4 β-amyloid peptide formation inhibitor provides a therapeutic benefit.

9. Compound of formula (I) according to any one of Claims 1 to 3, in the form of a pharmaceutically acceptable base, salt, hydrate or solvate, for use in the treatment of senile dementia, Alzheimer's disease, Down's syndrome, Parkinson's disease, amyloid angiopathy, cerebrovascular disorders, frontotemporal dementias and Pick's disease, post-traumatic dementias, pathologies linked to neuroinflammatory processes, Huntington's disease and/or Korsakov's syndrome.

## Patentansprüche

1. Verbindung der allgemeinen Formel (I): 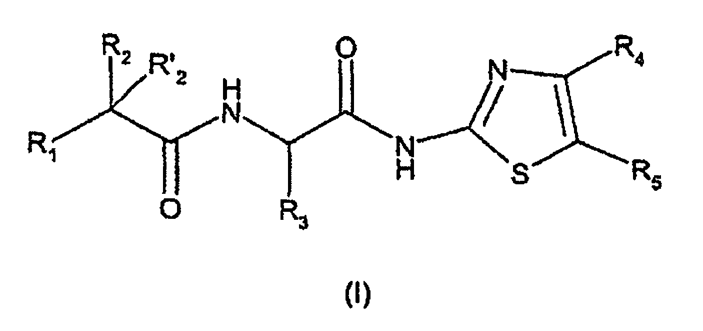 worin
R₁ für ein C₁₋₆-Alkyl, das gegebenenfalls durch einen bis drei Substituenten substituiert ist, die unter einem Halogen, einem Trifluormethyl, einem Hydroxy, einem C₁₋₆-Alkoxy, einem C₁₋₆-Thioalkyl, einem Thiophen oder einem Phenyl ausgewählt sind; oder ein C₃₋₇-Cycloalkyl, ein Thiophen, ein Benzothiophen, ein Pyridinyl, ein Furanyl oder ein Phenyl steht; wobei die Phenylgruppen gegebenenfalls durch einen bis drei Substituenten substituiert sind, die unter einem Halogenatom, einem C₁₋₆-Alkyl, einem C₁₋₆-Alkoxy, einem Hydroxy, einem Methylendioxy, einem Phenoxy, einem Benzyloxy oder einem Trifluormethyl ausgewählt sind;
R₂ und R'₂ unabhängig voneinander für ein Wasserstoffatom, ein Halogenatom, ein Hydroxy, ein C₁₋₃-Alkoxy, ein C₁₋₃-Alkyl, ein C₃₋₇-Cycloalkyl oder eine O-C (O) -C₁₋₆-Alkylgruppe stehen oder R₂ und R'₂ gemeinsam eine Oxogruppe bilden;
R₃ für ein Wasserstoffatom oder ein C₁₋₆-Alkyl, das gegebenenfalls durch ein Hydroxy, ein C₁₋₆-Cycloalkyl oder ein C₁₋₃-Alkoxy substituiert ist, steht; R₄ und R₅ unabhängig voneinander für ein Wasserstoffatom, ein C₁₋₇-Alkyl, ein Trifluormethyl, eine Gruppe L oder eine Gruppe Z stehen; 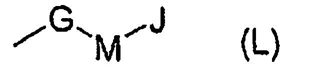 G für ein C₁₋₇-Alkyl oder eine Einfachbindung steht;
M für ein C₃₋₇-Cycloalkyl, ein Phenyl, ein Naphthyl oder ein Pyridinyl steht, wobei die Gruppe M gegebenenfalls durch eine oder mehrere Gruppen substituiert ist, die unter einem Halogenatom, einer Hydroxygruppe, einem C₁₋₃-Alkyl, einem C₁₋₃-Alkoxy, einem Trifluormethyl, einem Trifluormethoxy oder einem -O-CHF₂ ausgewählt sind;
J für ein Wasserstoffatom oder eine Gruppe -Y-K steht;
Y für eine Einfachbindung, ein Sauerstoff- oder Schwefelatom oder eine -C₁₋₄-Alkylen-, -O-C₁₋₄-Alkylen- oder -C₁₋₄-Alkylen-o-Gruppe oder eine Gruppe -N(W)- steht, wobei die -C₁₋₄-Alkylengruppe gegebenenfalls durch eine Hydroxy- oder C₁₋₃-Alkoxygruppe substituiert ist;
W für ein Wasserstoffatom, ein C₁₋₃-Alkyl, das gegebenenfalls durch ein Phenyl substituiert ist, oder ein Phenyl steht;
K für eine Phenyl- oder Pyridinylgruppe steht, wobei die Gruppe K gegebenenfalls durch eine oder mehrere Gruppen substituiert ist, die unter einem Halogenatom, einer Hydroxygruppe, einem C₁₋₃-Alkyl, einem C₁₋₃-Alkoxy, einem Trifluormethyl, einem Trifluormethoxy und einem -O-CHF₂ ausgewählt sind; mit der Maßgabe, daß mindestens eine Gruppe R₄ oder R₅ für eine Gruppe Z steht;
Z für eine CN-Gruppe, eine SO₂NR₆R₇-Gruppe oder eine heteroaromatische Gruppe steht; wobei die heteroaromatische Gruppe gegebenenfalls durch eine Gruppe R₈ substituiert ist; wobei R₈ für ein C₁₋₄-Alkyl, das gegebenenfalls selbst durch ein CN, ein Phenyl oder ein Phenoxy substituiert ist, oder ein Phenyl steht; wobei die Phenyl- und Phenoxygruppen gegebenenfalls durch 1 bis 3 Gruppen substituiert sind, die unter einem Halogenatom, einem C₁₋₃-Alkyl, einem C₁₋₃-Alkoxy und einem Trifluormethyl ausgewählt sind;
R₆ und R₇ unabhängig voneinander für ein Wasserstoffatom, eine C₁₋₆-Alkylgruppe, die gegebenenfalls durch ein C₃₋₇-Cycloalkyl, ein C₃₋₇-Cycloalkenyl, C₁₋₃-Alkoxy, ein Phenyl, ein Naphthalinyl, ein Morpholinyl oder ein Pyridinyl substituiert ist; oder ein C₃₋₇-Cycloalkyl, C₁₋₆-Alkoxy, ein Phenyl oder ein Indanyl stehen; wobei die C₃₋₇-Cycloalkyl-, C₃₋₇-Cycloalkenyl-, Phenyl-, Naphthalinyl-, Morpholinyl-, Pyridinyl- und Indanylgruppen gegebenenfalls durch eine oder zwei Gruppen substituiert sind, die unter einem C₁₋₃-Alkyl, einem Hydroxy, einem C₁₋₃-Alkoxy, einem Phenyl oder einem Halogenatom ausgewählt sind; oder
R₆ und R₇ mit dem Stickstoffatom, an das sie gebunden sind, einen Aziridin-, Azetidin-, Pyrrolidin-, Piperidin-, Morpholin- oder Benzopiperidinring bilden;
in Basen-, Säureadditionssalz-, Hydrat- oder Solvatform.

2. Verbindung nach Anspruch 1, **dadurch gekennzeichnet, daß**:
R₁ für ein C₁₋₆-Alkyl oder ein Phenyl, das gegebenenfalls durch ein bis drei Halogenatome substituiert ist, steht;
R₂ und R'₂ unabhängig voneinander für ein Wasserstoffatom oder ein Hydroxy stehen;
R₃ für ein C₁₋₆-Alkyl steht;
R₄ und R₅ unabhängig voneinander für ein Wasserstoffatom, ein C₁₋₇-Alkyl, ein Trifluormethyl, eine Gruppe L oder eine Gruppe Z stehen; 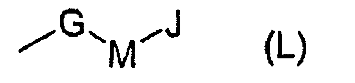
G für ein C₁₋₇-Alkyl oder eine Einfachbindung steht;
M für ein Phenyl, das gegebenenfalls durch ein oder mehrere Halogenatome substituiert ist, steht;
J für ein Wasserstoffatom oder eine Gruppe -Y-K steht;
Y für eine Einfachbindung, ein Sauerstoffatom oder -O-C₁₋₄-Alkylen- steht;
K für eine Phenylgruppe steht, die gegebenenfalls durch eine oder mehrere Gruppen substituiert ist, die unter einem Halogenatom, einem C₁₋₃-Alkyl und einem Trifluormethyl ausgewählt sind;
mit der Maßgabe, daß mindestens eine Gruppe R₄ oder R₅ für eine Gruppe Z steht;
Z für eine CN-Gruppe, eine SO₂NR₆R₇-Gruppe oder eine heteroaromatische Gruppe steht; wobei die heteroaromatische Gruppe gegebenenfalls durch eine Gruppe R₈ substituiert ist; wobei R₈ für ein C₁₋₄-Alkyl, das gegebenenfalls selbst durch ein Phenyl substituiert ist, oder ein Phenyl steht;
R₆ und R₇ unabhängig voneinander für ein Wasserstoffatom, eine C₁₋₆-Alkylgruppe, die gegebenenfalls durch ein Phenyl oder ein Naphthalinyl substituiert ist; oder ein Phenyl oder ein Indanyl stehen; wobei die Phenylgruppen gegebenenfalls durch eine oder zwei Gruppen substituiert sind, die unter einem C₁₋₃-Alkoxy, einem Phenyl oder einem Halogenatom ausgewählt sind; oder
R₆ und R₇ mit dem Stickstoffatom, an das sie gebunden sind, einen Benzopiperidinring bilden; in Basen-, Säureadditionssalz-, Hydrat- oder Solvatform.

3. Verbindung der Formel (I) nach Anspruch 1 oder 2, **dadurch gekennzeichnet, daß**:
R₁ für ein C₁₋₄-Alkyl, vorzugsweise ein Isopropyl oder ein tert.-Butyl, oder ein Phenyl, das durch zwei Fluoratome substituiert ist, steht;
R₂ und R'₂ unabhängig voneinander für ein Wasserstoffatom oder ein Hydroxy stehen;
R₃ für ein C₁₋₄-Alkyl, vorzugsweise ein Methyl, Ethyl oder Propyl, steht;
in Basen-, Säureadditionssalz-, Hydrat- oder Solvatform.

4. Verfahren zur Herstellung einer Verbindung der Formel (I) nach einem der Ansprüche 1 bis 3, bei dem man
eine Peptidkupplung des 2-Aminothiazols der Formel (III) 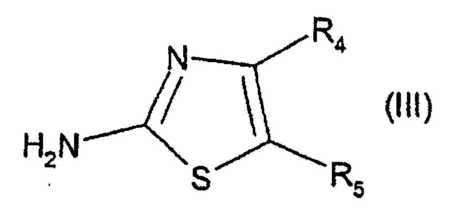 mit der Acylaminosäure der Formel (II) 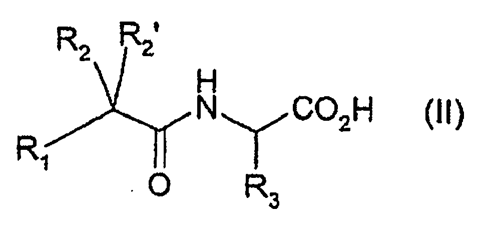 durchführt, wobei R₁, R₂ , R'₂, R₃, R₄ und R₅ die in Formel (I) nach einem der Ansprüche 1 bis 3 angegebene Bedeutung besitzen.

5. Verfahren zur Herstellung einer Verbindung der Formel (I) nach einem der Ansprüche 1 bis 3, bei dem man
eine Peptidkupplung der Verbindung der Formel (IV) 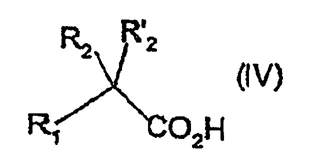 mit dem Amin der Formel (VI) 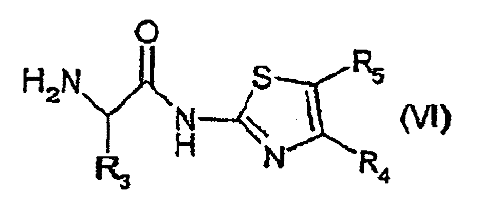 durchführt, wobei R₁, R₂, R'₂, R₃, R₄ und R₅ die in Formel (I) nach einem der Ansprüche 1 bis 3 angegebene Bedeutung besitzen.

6. Arzneimittel, **dadurch gekennzeichnet, daß** es eine Verbindung der Formel (I) nach einem der Ansprüche 1 bis 3 in pharmazeutisch unbedenklicher Basen-, Salz-, Hydrat- oder Solvatform umfaßt.

7. Pharmazeutische Zusammensetzung, enthaltend mindestens eine Verbindung der Formel (I) nach einem der Ansprüche 1 bis 3 in pharmazeutisch unbedenklicher Basen-, Salz-, Hydrat- oder Solvatform und gegebenenfalls einen oder mehrere pharmazeutisch unbedenkliche Hilfsstoffe.

8. Verbindung der Formel (I) nach einem der Ansprüche 1 bis 3 in pharmazeutisch unbedenklicher Basen-, Salz-, Hydrat- oder Solvatform zur Verwendung zur Behandlung einer Pathologie, bei der ein Inhibitor der Bildung des β-Amyloidpeptids β-A4 einen therapeutischen Nutzen erbringt.

9. Verbindung der Formel (I) nach einem der Ansprüche 1 bis 3 in pharmazeutisch unbedenklicher Basen-, Salz-, Hydrat- oder Solvatform zur Verwendung zur Behandlung von seniler Demenz, Alzheimer-Krankheit, Down-Syndrom, Parkinson-Krankheit, Amyloidangiopathie, zerebrovaskulären Störungen, frontotemperoralen Demenzen und Pick-Krankheit, posttraumatischen Demenzen, Pathologien in Verbindung mit Neuroinflammationsprozessen, Chorea Huntington und/oder Korsakow-Syndrom.
